# EUROPEAN PATENT APPLICATION

(11) **EP 3 957 650 A1**
(43) Date of publication of application: **23.02.2022**
(21) Application number: 20191286.2
(22) Date of filing: 17.08.2020
(51) Int. Cl.: C07K 14/525, C07K 14/78, A61K 35/12

(54) **CO-STIMULATORY 4-1BBL ECTODOMAIN POLYPEPTIDES FOR IMMUNOMODULATION**

(71) Applicant: Themis Bioscience GmbH, 1190 Vienna (AT)
(72) Inventor: STRITZKER, Jochen, 3400 Kierling (AT); STEINBERGER, Peter, 1170 Vienna (AT); LUPINEK, Daniela, 1230 Vienna (AT); DE SOUSA LINHARES, Annika, 3452 Michelndorf (AT)
(74) Representative: Eisenführ Speiser

(57) **Abstract**

The present invention generally relates to the field of immunomodulation and provides various costimulatory polypeptides comprising at least one 4-1BBL ectodomain comprising a minimal TNF Homology Domain. Further provided are vectors and oncolytic viruses expressing the 4-1BBL ectodomain and methods for producing the same. Additionally provided are methods and uses for obtaining and optionally purifying the inherently oligomerizing 4-1 BBL ectodomain polypeptides and associated oncolytic viruses for highly targeted cancer treatment, including the treatment of tumors. Finally, there are provided kits and uses of the polypeptides and vectors as disclosed.

## Description

### Technical Field

The present invention relates to the field of immunomodulation and provides various costimulatory polypeptides comprising at least one 4-1BBL ectodomain comprising a minimal TNF Homology Domain. Further provided are vectors and oncolytic viruses expressing the 4-1BBL ectodomain polypeptides and methods for producing the same. Additionally provided are methods and uses for obtaining and optionally purifying the inherently oligomerizing 4-1 BBL ectodomain polypeptides and associated oncolytic viruses for highly targeted cancer treatment. Finally, kits are provided and uses of the polypeptides and vectors are disclosed.

### Background

Cancer immunotherapy is becoming of increasing interest for providing new cancer treatments. Specifically targeting cancerous cells by immune-oncolytic strategies emerges as a highly promising therapeutic way to obtain durable disease remission. Even though the immunotherapy is steadily changing the established treatment paradigms for cancer therapy and expanding the treatment options, the technology is still in its infancy and new improvements applicable to the clinic are needed.

Cancer immunotherapy generally involves or uses components of the immune system to attack cancerous cells in a patient's body and does not rely on the sole use of usually harsh chemotherapeutics, radiation etc. which often have severe side effects for a patient. Cancer immunotherapy approaches often aim at enhancing endogenous immune responses of a patient towards tumor-associated antigens (TAAs). Tumor antigens are usually divided in three classes. TAAs, tumor specific antigens (TSAs), and cancer germline antigens (CGAs). Whilst TSAs are only expressed in cancer cells (and not in healthy tissue), TAAs correspond to antigens usually expressed in healthy tissue/on healthy cells at low levels, but are overexpressed by certain tumor cells. In particular monoclonal antibodies (mAbs) have been the major tools to target TSAs and TAAs in cancer treatment in ongoing clinical trials over the last decades. Finally, CGAs are expressed in tumor cells of different histological origins, but they are silent in most normal adult cells.

In immunotherapy, an effective immune response requires two types of biological signal to fully activate T cells. The first signal, namely antigen-specific signal, is generated by the interaction of lymphocyte receptor with specific peptides bound to major histocompatibility complexes (MHC) molecules on antigen-presenting cells (APCs). The second signal is a co-stimulation signal, and is not antigen specific. This type of signal is provided upon interaction of costimulatory receptors expressed on T cells with their respective ligands expressed on APCs.

Strengthening the response of (endogenous) tumor-specific T cells is a viable strategy to combat cancers. To this end, these T-cells, but also other relevant immune effectors, including natural killer (NK) cells have to be efficiently stimulated via the relevant co-stimulatory complexes or molecules to present the second signal introduced above.

Thereby, it is long known that oligomerization of tumor necrosis family ligands such as CD40L is required for triggering of signaling pathways. Similarly, the membrane-bound form of Fas ligand (FasL) signals apoptosis in target cells through engagement of the death receptor Fas, while the proteolytically processed, soluble form of FasL does not induce cell death. Nevertheless, soluble FasL can be rendered active upon cross-linking and the oligomerization of FasL, is required for triggering of the signaling pathways (Holler et al, Molecular and Cellular Biology, Feb. 2003, p. 1428-1440.). Holler et al. used the collagen domain of ACRP30 for achieving an oligomerization *in vitro.* Surprisingly however, the collagen domain did not result in trimerization but rather in a dimerization of FasL trimers.

More recently, specific co-stimulation of activated T-cells, NK cells, and other immune cells via the tumor necrosis factor receptor superfamily (TNFRSF) member 4-1BB (CD137) has emerged as a promising approach for cancer immunotherapy (Claus et al., Sci. Transl. Med. 11, eaav5989 (2019)). 4-1BB is a type 1 transmembrane glycoprotein receptor belonging to the TNF superfamily, expressed on certain immune cells. 4-1BB/CD137 expression is very selective and can be found, e.g. on activated T Lymphocytes and NK cells.

4-1BBL (abbreviation for: 4-1BB ligand, alternative name: CD137L) can e.g. be found on antigen-presenting cells (APCs) and is an agonistic binder of 4-1BB and therefore an interesting protein to stimulate T cells and other immune cells expressing 4-1BB. However, when using 4-1BBL in therapeutic settings, it has to be considered that the extracellular, non-cell associated 4-1BBL has very low activity as monomer.

Different strategies have been used to overcome this problem. First, agonistic 4-1BB antibodies (e.g. utomilumab (humanized IgG2 mAb), urelumab (human IgG4 mAb)) were designed (Chu et al., 2019, Int. J. Mol. Sci., 20). The above mAbs, however, were reported to result in severe toxicity and inflammation. Agonistic antibodies, for example, were shown to have significant systemic toxicity (e.g. urelumab), or relatively low efficacy (e.g. utomilumab) so that they never progressed beyond clinical trials (Claus et al., *supra*)*.* Clustering of e.g. urelumab achieved through Fc gamma R-binding, is associated with liver toxicity (Claus et al., *supra*)*.* One way to reduce systemic toxicities is to administer 4-1BB specific antibodies intratumorally (Chester et al., 2018, Blood, 131, 49-57) or an approach where "masked" antibodies become "unmasked" by tumor-specific protease activity, which was also reported by Chester et al.

Some cancer immunotherapies, for example, rely on artificial functional fragments of Abs, e.g. single-chain variable fragments (scFv) that may target TAAs/TSAs/CGAs in a highly specific manner. WO 2012/049328 A1 and WO 2019/234187 A1 disclose an engineered scFv fusion polypeptide based on the fusion of antibody-derived binding domains to a homotrimerization region, which yields trimeric scFv. Still, this trimerization of scFv-fragments antibody formats, or of single-domain antibody (sdAb) polypeptides via a trimerization domain has the disadvantage of having a dependence on the activity of each scFv-polypeptide as an Ab-derived recognition moiety being a non-natural molecule for the immune system of a patient. Therefore, scFv fragments and other Ab-derived polypeptides can comprise protein sequences recognized as "foreign" by the immune system, which could lead to the formation of a drug-specific immune response.

Claus et al. *supra* use an alternative strategy in which three individual 4-1BBL ectodomains are fused to an immunoglobulin (Ig)-like protein, which also recognizes TAAs (i.e. FAP) for targeting of malignant cells. This approach, however, may not overcome undesired effects associated with the use of antibody-derived sequences.

Therefore, there is an increasing need in using naturally occurring, agonistic ligand sequences and a fully human or humanized system to avoid severe side effects for patients.

Another recent strategy in immuno-oncology is the adoptive transfer of specifically modified T-cells (chimeric antigen receptor (CAR)-modified T-cells), in particular, for patients that suffer from hematologic malignancies like leukemia and lymphoma. Zou and Jianyong (Journal of Hematology & Oncology, (2018) 11:130), for example, report a CAR-T-cell equipped with CD137 to test new chronic lymphocytic leukemia (CLL) therapies by providing a T-cell intracellular co-stimulatory signal via CD137. Still, CAR-T-cell therapy encounters multiple challenges when used to treat solid tumors, including the immunosuppressive tumor microenvironment and heterogeneity of antigen expression which can presently not be solved by the use of CAR-T-cells as monotherapy. Further, the CAR T-cell-mediated therapy field embraced the challenge of applying this approach to treat common epithelial malignancies, which make up the majority of cancer cases, but evade immunologic attack by a variety of subversive mechanisms (Srivastava and Riddell, J. Immunol., 200(2): 459-468, 2018).

Another promising cancer immunotherapy in addition to Ab- and CAR-T-cell-based strategies relies on oncolytic viruses (OVs) that preferentially infect and kill cancer cells in a highly specific manner. OV-based cancer treatment is based on the selection of viruses having an intrinsic tropism for tumor cells, e.g. by binding to receptors that are (over)expressed on tumors and/or replicate selectively/preferentially in tumor cells. These viral vectors can thereby either directly kill infected tumor cells or increase their susceptibility to cell death and/or apoptosis. Beside the primary effect of tumor lysis, OVs can additionally stimulate the immune system to attack malignant cells. Tumors are usually an immuno-suppressive environment in which the immune system is silenced in order to avoid the immune response against the cancer cells. OVs thus may help to break this immune suppression to achieve a durable immune response against specific structures on tumor cells. Further, OVs can be specifically equipped with payloads to break checkpoints and/or to transform cold into hot tumors.

Another suggested technology reported for targeting 4-1BB is the use of 4-1BB-bispecific aptamer complexes, which are composed of an agonistic 4-1BB oligonucleotide aptamer conjugated to an aptamer that binds prostate-specific membrane antigen (PSMA) or vascular endothelial growth factor (Schrand et al., Cancer Immunol. Res., 2, 867-77, 2014).

Bispecific DART^{®} molecules - polypeptides of PD-L1/4-1BB fused antibodies - are also in development. DARTs activate 4-1BB when they are "multimerized" on the cell surface via binding to PD-L1 (Berezhnoy et al, MacroGenics, Presented at the 30th EORTC/AACR/NCI Symposium, November 13-16, 2018, Dublin, Ireland, Abstract 216, PB-067).

In another example, 4-1BBL trimerization or hexamerization could stimulate 4-1BB activation and was obtained by using Tenascin-C (TNC) and flag-antibodies (Wyzgol et al., K. Immunol., 2009, 183, 1851-61).

Other groups then tried to fuse the extracellular domains of 4-1BBL to the C-terminus of a modified form of core streptavidin (SA) resulting in SA-4-1 BBL. The SA-4-1BBL molecule forms tetramers/oligomers, owing to the structural features of SA, and has the ability to cross-link 4-1 BB receptors for potent costimulatory activity on T-cells. To this end, a specific 4-1BBL domain (amino acids (aa) 104-309) was used (Schabowsky et al., Vaccine, 2009, 28, 512-522).

From other groups, an AviTag-4-1BBL polypeptide was designed (Rabu et al., The Journal of Biological Chemistry, vol. 280, no. 50, pp. 41472-41481). The AviTag binds biotin which can then be used for fusion to (strept)avidin. A TNF-homology-domain, as such also comprising the N-terminal tail sequence (and thus a cysteine at position C51 within the extracellular domain of 4-1BBL) resulted in trimerization of 4-1BBL, which was then further multimerized by binding to (strept)avidin.

Generally speaking, 4-1BBL may have better efficacy and safety compared to Abs by delivering signals quantitatively and/or qualitatively different from those transduced by Abs. In general, full-length antibody-based approaches frequently result in antibody-dependent cellular toxicity, which is obviously deleterious when co-stimulatory effects are desired.

The currently known strategies to introduce 4-1BB costimulatory signals to enhance immune responses against tumors have disadvantages:
- A TNC chicken protein as used in Wyzgol et al. *supra* may favor trimerization, but it may cause a severe immune response against this chicken protein payload when applied to humans, as this portion will be recognized as foreign by the human immune system. In addition, TNC-trimerization alone does not seem to be optimal to sufficiently stimulate 4-1BB clustering and activation in functional cell culture assays.
- (Strept)Avidin as cross linker of 4-1BBL has the major disadvantage of being a bacterial protein against which an immune response might be triggered that would strongly reduce therapeutic efficacy.
- For the AviTag-approach the assembly process may represent an additional complication, as it requires both, purified AviTag-containing proteins as well as purified (strept)avidins. From a stoichiometry point of view, this cannot be easily maintained in an *in vivo* setting in a living organism.
- Specifically, regarding the use of aptamers, their clinical benefit still has to be demonstrated and successful tumor targeting strategies *in vivo* are missing to date.
- The approach detailed by Claus et al., *supra,* relies on a protein encoded by this approach that is much larger than in nature and targeting is dependent on a single molecule (i.e. a tumor associated antigen such as HER2), which might not be present in every tumor tissue within the patient, e.g. in metastases.
- The PD-L1/4-1BB DART approach as disclosed in Berezhnoy et al., *supra,* can be problematic since a) not all cancer cells overexpress PD-L1 and b) numerous other healthy cells express PD-L1. Therefore, targeting the tumor is not guaranteed. Moreover, combinations with approved PD-L1 inhibitors might be problematic, as healthy cells expressing PD-L1 would also be affected, leading to unwanted toxic side effects.
- Intratumoral injection of proteins is a problematic approach in clinical settings since the concentration of the injected protein constantly decreases and its half-life time cannot be determined reliably. Furthermore, with this approach T cells are not recruited into the tumor tissue.
- Finally, protease-based techniques have the disadvantage that they might not be expressed in all malignant tissues as the absence of T cells in cold tumors places the active molecule away from target cells.

Additionally, all presently available non-Ab-based approaches targeting 4-1BB suffer from the inherent drawback that the use of non-human sequences in a potential therapeutic is necessary. These approaches are thus always confronted with the problem that undesired and partly severe side effects as caused by the immune system and as such hard to predict may arise so that there is an ongoing need to identify safer strategies for immuno-oncology.

Therefore, the 4-1BB activating molecules designed so far may function well in *in vitro* settings, but they all are associated with major disadvantages when it comes to the use as safe and effective therapeutics in patients.

Another problem associated with the various 4-1BB activating molecules designed so far is the fact that the molecule as such can cause unwanted toxicities if it cannot be targeted to the relevant tumor, or to the site of interest in a patient's body in functional, i.e., usually trimerized, state.

An additional immunotherapeutic strategy is the use of oncolytic viruses as promising strategy against cancer, including solid tumors. Oncolytic viruses (OVs), including vaccinia virus, myxoma virus, adenovirus, herpes virus, reovirus, Zika virus, vesicular stomatitis virus, parvovirus, poliovirus, influenza virus, arenavirus, coxsackie virus, semliki forest virus, sindbis virus, maraba virus, seneca valley virus, Newcastle disease virus, mumps virus, and measles virus, including 1957 Leningrad-derived strains, for example Leningrad-4, and Cangchun-47, 1960 Shanghai-derived strains, for example Shanghai-191, 1968 Tanabe-derived strains, such as CAM-70, as well as Edmonston-derived strains, for example, Edmonston Seed "B", AIK-C, Moraten, Schwarz, Rubeovax, and Zagreb, can efficiently target cancer cells, but not normal cells, leading to lysis of the tumor mass. OVs derived from attenuated viruses are usually *per se* safe for use as vaccines or therapeutics. Beside this primary effect, OVs can also stimulate the immune system. Tumors are an immuno-suppressive environment in which the immune system is silenced in order to avoid the immune response against cancer cells. The delivery of OVs into the tumor activates the immune system so that it can facilitate a strong and durable response against the tumor itself. Both innate and adaptive immune responses contribute to this process, producing an immune response against tumor antigens and facilitating immunological memory (Marelli et al., Front. Immunol. 2018; 9:866). Further, an OV of interest can be equipped with different payloads of interest to optimize the anti-tumor response. Further, OVs can be used together with other therapeutics including immunomodulatory molecules such as checkpoint inhibitors. Checkpoint inhibitors are a class of molecules blocking the negative regulators of T-cell function (immune checkpoints), thereby increasing T-cell activation to enhance cancer immunotherapy in a targeted way (LaRocca and Warner, Clin. Transl. Med., 2018; 7:35).

It was thus an objective of the present invention to provide novel polypeptides with 4-1BB stimulating activity that are designed to address known features of 4-1BB activation. This includes necessary clustering of the activated receptor but shows reduced toxicity in comparison to known agonistic antibodies and is able to induce a signal that is qualitatively equal to the natural ligand. In particular, a functional 4-1BBL in multimeric form should be designed that uses human-derived sequences only to reduce the risk of side-effects and anti-drug immune responses.

Moreover, it was an objective to identify tumor-specific expression strategies for the 4-1BB stimulating molecules to further reduce the chance of systemic toxicity and simultaneously to enhance a tumor-specific T cell response by providing *in situ* expression and functional assembly of the 4-1BB stimulating molecules overcoming the problems as presently associated with 4-1BB cargos. To this end, new strategies for the treatment of cancer should be established. Finally, it was an object to provide a strategy for the enhanced recruitment of T-cells into a tumor tissue, i.e. to break immuno-tolerance in certain tumor tissues and to induce a targeted anti-tumor inflammatory response.

### Summary of the Invention

To address the above objects, in a first aspect, there is thus provided a costimulatory polypeptide comprising (a) (i) at least one 4-1BBL ectodomain and a trimerization domain, and/or (ii) at least one 4-1BBL ectodomain and at least one leader sequence, and optionally at least one affinity tag; wherein each 4-1BBL ectodomain comprises or consists of the minimal Tumor Necrosis Factor (TNF) Homology Domain according to SEQ ID NO: 1, or a sequence having at least 90%, 91%, 92%, 93%, 94%, 95%, 96%, 97%, 98% or 99% sequence identity to SEQ ID NO: 1, or a nucleic acid sequence encoding the polypeptide ; and/or (b) at least three 4-1BBL ectodomains, and optionally comprising at least one trimerization domain, wherein each 4-1BBL ectodomain comprises or consists of the minimal TNF Homology Domain according to SEQ ID NO: 1, or a sequence having at least 90%, 91%, 92%, 93%, 94%, 95%, 96%, 97%, 98% or 99% sequence identity to SEQ ID NO: 1, or a nucleic acid sequence encoding the polypeptide ; and wherein the 4-1BBL ectodomain does not comprise the cysteine residue at position 51 of the naturally occurring human 4-1BBL ectodomain as shown in SEQ ID NO: 2, or a nucleic acid sequence encoding the same; and wherein the polypeptide binds to 4-1BB on the surface of a 4-1BB expressing cell and thus triggers 4-1BB-mediated immune cell stimulation.

In specific embodiments of this first aspect, the trimerization domain of any of the preceding costimulatory polypeptides is selected from SEQ ID NO: 7 or 8.

In one embodiment of the above aspect, the at least one 4-1BBL ectodomain of the costimulatory polypeptide comprises or consists of a sequence according to SEQ ID NOs: 3 to 6, or a polypeptide with a sequence having at least 90%, 91%, 92%, 93%, 94%, 95%, 96%, 97%, 98% or 99% sequence identity to the respective sequence, or a nucleic acid sequence encoding the same. In specific embodiments, the at least one 4-1BBL ectodomain of the costimulatory polypeptide comprises the amino acid sequence of SEQ ID NO: 3, or a polypeptide with a sequence having at least 90%, 91%, 92%, 93%, 94%, 95%, 96%, 97%, 98% or 99% sequence identity to SEQ ID NO: 3, or a nucleic acid sequence encoding the same. In specific embodiments, the at least one 4-1BBL ectodomain of the costimulatory polypeptide comprises SEQ ID NO: 4, or a polypeptide with a sequence having at least 90%, 91%, 92%, 93%, 94%, 95%, 96%, 97%, 98% or 99% sequence identity to SEQ ID NO: 4, or a nucleic acid sequence encoding the same. In specific embodiments, the at least one 4-1BBL ectodomain of the costimulatory polypeptide comprises SEQ ID NO: 5, or a polypeptide with a sequence having at least 90%, 91%, 92%, 93%, 94%, 95%, 96%, 97%, 98% or 99% sequence identity to SEQ ID NO: 5, or a nucleic acid sequence encoding the same. In specific embodiments, the at least one 4-1BBL ectodomain of the costimulatory polypeptide comprises SEQ ID NO:6, or a polypeptide with a sequence having at least 90%, 91%, 92%, 93%, 94%, 95%, 96%, 97%, 98% or 99% sequence identity to SEQ ID NO: 6, or a nucleic acid sequence encoding the same.

In another embodiment of the above aspect, is any one of the preceding costimulatory polypeptides or embodiments thereof, wherein the polypeptide comprises at least one linker. In specific embodiments, the at least one linker comprises or consists of a sequence individually selected from the group consisting of SEQ ID NOs: 9 to 16, or a sequence having at least 90%, 91%, 92%, 93%, 94%, 95%, 96%, 97%, 98% or 99% sequence identity to any one of the sequences SEQ ID NOs: 9 to 16, or a nucleic acid sequence encoding the same. In specific embodiments, the at least one linker comprises or consists of the amino acid sequence of SEQ ID NO: 9, or a sequence having at least 90%, 91%, 92%, 93%, 94%, 95%, 96%, 97%, 98% or 99% sequence identity to SEQ ID NO: 9, or a nucleic acid sequence encoding the same. In specific embodiments, the at least one linker comprises or consists of the amino acid sequence of SEQ ID NO: 10, or a sequence having at least 90%, 91%, 92%, 93%, 94%, 95%, 96%, 97%, 98% or 99% sequence identity to SEQ ID NO: 10, or a nucleic acid sequence encoding the same. In specific embodiments, the at least one linker comprises or consists of the amino acid sequence of SEQ ID NO: 11, or a sequence having at least 90%, 91%, 92%, 93%, 94%, 95%, 96%, 97%, 98% or 99% sequence identity to SEQ ID NO: 11, or a nucleic acid sequence encoding the same. In specific embodiments, the at least one linker comprises or consists of the amino acid sequence of SEQ ID NO: 12, or a sequence having at least 90%, 91%, 92%, 93%, 94%, 95%, 96%, 97%, 98% or 99% sequence identity to SEQ ID NO: 12, or a nucleic acid sequence encoding the same. In specific embodiments, the at least one linker comprises or consists of the amino acid sequence of SEQ ID NO: 13, or a sequence having at least 90%, 91%, 92%, 93%, 94%, 95%, 96%, 97%, 98% or 99% sequence identity to SEQ ID NO: 13, or a nucleic acid sequence encoding the same. In specific embodiments, the at least one linker comprises or consists of the amino acid sequence of SEQ ID NO: 14, or a sequence having at least 90%, 91%, 92%, 93%, 94%, 95%, 96%, 97%, 98% or 99% sequence identity to SEQ ID NO: 14, or a nucleic acid sequence encoding the same. In specific embodiments, the at least one linker comprises or consists of the amino acid sequence of SEQ ID NO: 15, or a sequence having at least 90%, 91%, 92%, 93%, 94%, 95%, 96%, 97%, 98% or 99% sequence identity to SEQ ID NO: 15, or a nucleic acid sequence encoding the same. In specific embodiments, the at least one linker comprises or consists of the amino acid sequence of SEQ ID NO: 16, or a sequence having at least 90%, 91%, 92%, 93%, 94%, 95%, 96%, 97%, 98% or 99% sequence identity to SEQ ID NO: 16, or a nucleic acid sequence encoding the same.

In specific embodiments of the above aspects or any one of the preceding costimulatory polypeptides or embodiments thereof, the costimulatory polypeptide, or the sequence encoding the same, additionally comprises at least one affinity tag and/or at least one protease cleavage tag and/or at least one inhibitory domain and/or at least one leader sequence or nucleic acid encoding the same. In specific embodiments, the costimulatory polypeptide, or the sequence encoding the same, additionally comprises at least one affinity tag or nucleic acid encoding the same. In specific embodiments, the costimulatory polypeptide, or the sequence encoding the same, additionally comprises at least one protease cleavage tag or nucleic acid encoding the same. In specific embodiments, the costimulatory polypeptide, or the sequence encoding the same, additionally comprises at least one inhibitory domain or nucleic acid encoding the same. In specific embodiments, the costimulatory polypeptide, or the sequence encoding the same, additionally comprises at least one leader sequence or nucleic acid encoding the same.

In specific embodiments, the costimulatory polypeptide, or the sequence encoding the same, additionally comprises at least one affinity tag or nucleic acid encoding the same and at least one protease cleavage tag or nucleic acid encoding the same. In specific embodiments, the costimulatory polypeptide, or the sequence encoding the same, additionally comprises at least one affinity tag or nucleic acid encoding the same and at least one inhibitory domain or nucleic acid encoding the same. In specific embodiments, the costimulatory polypeptide, or the sequence encoding the same, additionally comprises at least one affinity tag or nucleic acid encoding the same and at least one leader sequence or nucleic acid encoding the same. In specific embodiments, the costimulatory polypeptide, or the sequence encoding the same, additionally comprises at least one protease cleavage tag or nucleic acid encoding the same and at least one inhibitory domain or nucleic acid encoding the same. In specific embodiments, the costimulatory polypeptide, or the sequence encoding the same, additionally comprises at least one protease cleavage tag or nucleic acid encoding the same and at least one leader sequence or nucleic acid encoding the same. In specific embodiments, the costimulatory polypeptide, or the sequence encoding the same, additionally comprises at least one inhibitory domain or nucleic acid encoding the same and at least one leader sequence or nucleic acid encoding the same. In specific embodiments, the costimulatory polypeptide, or the sequence encoding the same, additionally comprises at least one affinity tag or nucleic acid encoding the same, at least one protease cleavage tag or nucleic acid encoding the same, and at least one inhibitory domain or nucleic acid encoding the same. In specific embodiments, the costimulatory polypeptide, or the sequence encoding the same, additionally comprises at least one affinity tag or nucleic acid encoding the same, at least one protease cleavage tag or nucleic acid encoding the same, and at least one leader sequence or nucleic acid encoding the same. In specific embodiments, the costimulatory polypeptide, or the sequence encoding the same, additionally comprises at least one affinity tag or nucleic acid encoding the same, at least one inhibitory domain or nucleic acid encoding the same, and at least one leader sequence or nucleic acid encoding the same. In specific embodiments, the costimulatory polypeptide, or the sequence encoding the same, additionally comprises at least one affinity tag or nucleic acid encoding the same, at least one protease cleavage tag or nucleic acid encoding the same, at least one inhibitory domain or nucleic acid encoding the same, and at least one leader sequence or nucleic acid encoding the same.

In yet another embodiment of the above aspect, is any one of the preceding costimulatory polypeptides or embodiments thereof, wherein the costimulatory polypeptide is encoded by a nucleic acid sequence selected from the group consisting of SEQ ID NOs: 47, 49, 51, 53, 55, 57, 59, 61, 63, 65, 67, 69, 71, 73, 75, 77 or 79 or a sequence having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to any one of the sequences SEQ ID NOs: 47, 49, 51, 53, 55, 57, 59, 61, 63, 65, 67, 69, 71, 73, 75, 77 or 79. In a specific embodiment, the costimulatory polypeptide is encoded by a nucleic acid sequence of SEQ ID NO: 47 or a sequence having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NO: 47. In a specific embodiment, the costimulatory polypeptide is encoded by a nucleic acid sequence of SEQ ID NO: 49 or a sequence having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NO: 49. In a specific embodiment, the costimulatory polypeptide is encoded by a nucleic acid sequence of SEQ ID NO: 51 or a sequence having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NO: 51. In a specific embodiment, the costimulatory polypeptide is encoded by a nucleic acid sequence of SEQ ID NO: 53 or a sequence having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NO: 53. In a specific embodiment, the costimulatory polypeptide is encoded by a nucleic acid sequence of SEQ ID NO: 55 or a sequence having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NO: 55. In a specific embodiment, the costimulatory polypeptide is encoded by a nucleic acid sequence of SEQ ID NO: 57 or a sequence having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NO: 57. In a specific embodiment, the costimulatory polypeptide is encoded by a nucleic acid sequence of SEQ ID NO: 59 or a sequence having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NO: 59. In a specific embodiment, the costimulatory polypeptide is encoded by a nucleic acid sequence of SEQ ID NO: 61 or a sequence having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NO: 61. In a specific embodiment, the costimulatory polypeptide is encoded by a nucleic acid sequence of SEQ ID NO: 63 or a sequence having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NO: 63. In a specific embodiment, the costimulatory polypeptide is encoded by a nucleic acid sequence of SEQ ID NO: 65 or a sequence having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NO: 65. In a specific embodiment, the costimulatory polypeptide is encoded by a nucleic acid sequence of SEQ ID NO: 67 or a sequence having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NO: 67. In a specific embodiment, the costimulatory polypeptide is encoded by a nucleic acid sequence of SEQ ID NO: 69 or a sequence having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NO: 69. In a specific embodiment, the costimulatory polypeptide is encoded by a nucleic acid sequence of SEQ ID NO: 71 or a sequence having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NO: 71. In a specific embodiment, the costimulatory polypeptide is encoded by a nucleic acid sequence of SEQ ID NO: 73 or a sequence having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NO: 73. In a specific embodiment, the costimulatory polypeptide is encoded by a nucleic acid sequence of SEQ ID NO: 75 or a sequence having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NO: 75. In a specific embodiment, the costimulatory polypeptide is encoded by a nucleic acid sequence of SEQ ID NO: 77 or a sequence having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NO: 77. In a specific embodiment, the costimulatory polypeptide is encoded by a nucleic acid sequence of SEQ ID NO: 79 or a sequence having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NO: 79.

In a second aspect, there is provided a vector comprising a nucleic acid sequence encoding a costimulatory polypeptide according to any one of the preceding costimulatory polypeptides of the first aspect or embodiments thereof.

In one embodiment of the above aspect, the vector comprises a sequence encoding an oncolytic virus, capable of expressing the costimulatory polypeptide. In specific embodiments, the oncolytic virus is selected from the group consisting of a vaccinia virus, a myxoma virus, an adenovirus, a herpes virus, a reovirus, a Zika virus, a vesicular stomatitis virus, a parvovirus, a poliovirus, an influenza virus, an arenavirus, a coxsackie virus, a semliki forest virus, a sindbis virus, a maraba virus, a seneca valley virus, a Newcastle disease virus, a mumps virus, and a measles virus, including 1957 Leningrad-derived strains, for example Leningrad-4, and Cangchun-47, 1960 Shanghai-derived strains, for example Shanghai-191, 1968 Tanabe-derived strains, such as CAM-70, as well as Edmonston-derived strains, for example, Edmonston See "B", AIK-C, Moraten, Schwarz, Rubeovax, and Zagreb, or combinations or a chimera thereof.

In another embodiment of the above aspect, there is provided an oncolytic virus encoded by a vector according to the second aspect or embodiments thereof, wherein the oncolytic virus comprises a costimulatory polypeptide according to the first aspect of the present invention. The encoded costimulatory polypeptide functions as the payload.

In a third aspect, there is provided a method of producing a costimulatory polypeptide , the method comprising: (i) providing a vector according to the above second aspect and embodiments thereof encoding a costimulatory polypeptide, (ii) introducing the vector into a host cell; (iii) culturing the host cell under conditions suitable for expression of the costimulatory polypeptide; (iv) optionally: isolating and purifying the costimulatory polypeptide ; and (v) obtaining the costimulatory 4-1BBL polypeptide .

In a fourth aspect, there is provided a method of producing an oncolytic virus expressing a costimulatory polypeptide, the method comprising: (i) providing a vector as defined according to the second aspect and embodiments thereof encoding an oncolytic virus comprising the costimulatory polypeptide, (ii) introducing the vector into a host cell, (iii) culturing the host cell under conditions suitable for expression and thus replication of the oncolytic virus, (iv) optionally: rescuing the oncolytic virus; (v) optionally: purifying the oncolytic virus as obtained in step (iii) or (iv); and (vi) obtaining an oncolytic virus.

In a fifth aspect, there is provided a pharmaceutical composition comprising a costimulatory polypeptide according to the first aspect and embodiments thereof as defined above and/or an oncolytic virus according to the second aspect and embodiments thereof as defined above, and/or as obtained according to the fourth aspect of the present invention as defined above.

In a sixth aspect of the present invention, there is provided a costimulatory polypeptide according to the first aspect and embodiments thereof as defined above, and/or an oncolytic virus according to the second aspect and embodiments thereof as defined above, and/or as obtained according to the fourth aspect as defined above for use in a method of treating cancer.

There is thus provided a method of treating a cancer in a subject by providing a costimulatory polypeptide and/or an oncolytic virus expressing a costimulatory polypeptide of the present invention. In a specific embodiment, the method of treating a cancer in a subject comprises providing a costimulatory polypeptide. In a specific embodiment, the method of treating a cancer in a subject comprises providing an oncolytic virus expressing a costimulatory polypeptide. In a specific embodiment, the method of treating a cancer in a subject comprises providing a first costimulatory polypeptide and providing an oncolytic virus expressing a second costimulatory polypeptide. In a specific embodiment, the first costimulatory polypeptide and the second costimulatory polypeptide have the same sequence. In a specific embodiment, the first costimulatory polypeptide and the second costimulatory polypeptide have different sequences.

Further provided is the use of an effective amount of costimulatory polypeptide and/or an effective amount of oncolytic virus expressing a costimulatory polypeptide of the present invention in a method of treating cancer in a subject in need thereof. In a specific embodiment, the disclosure provides use of an effective amount of costimulatory polypeptide of the present invention in a method of treating cancer in a subject in need thereof. In a specific embodiment, the disclosure provides use of an effective amount of oncolytic virus expressing a costimulatory polypeptide of the present invention in a method of treating cancer in a subject in need thereof. In a specific embodiment, the disclosure provides use of an effective amount of first costimulatory polypeptide of the invention and an effective amount of oncolytic virus expressing an effective amount of second costimulatory polypeptide in a method of treating cancer in a subject in need thereof. In a specific embodiment, the first costimulatory polypeptide and the second costimulatory polypeptide have the same sequence. In a specific embodiment, the first costimulatory polypeptide and the second costimulatory polypeptide have different sequences.

Further provided is use of an effective amount of costimulatory polypeptide and/or an effective amount of oncolytic virus expressing a costimulatory polypeptide of the present invention in the manufacture of a medicament for the treatment of cancer in a subject in need thereof. In a specific embodiment, the disclosure provides use of an effective amount of costimulatory polypeptide of the present invention in the manufacture of a medicament for the treatment of cancer in a subject in need thereof. In a specific embodiment, the disclosure provides use of an effective amount of oncolytic virus expressing a costimulatory polypeptide of the present invention in the manufacture of a medicament for the treatment of cancer in a subject in need thereof. In a specific embodiment, the disclosure provides use of a first costimulatory polypeptide of the invention and an oncolytic virus expressing a second costimulatory polypeptide in the manufacture of a medicament for the treatment of cancer in a subject in need thereof. In a specific embodiment, the first costimulatory polypeptide and the second costimulatory polypeptide have the same sequence. In a specific embodiment, the first costimulatory polypeptide and the second costimulatory polypeptide have different sequences.

In one embodiment of the foregoing: (i) methods of treating cancer, (ii) uses in a method of treating cancer, or (iii) uses in the manufacture of a medicament for treating cancer, the cancer is selected from bladder cancer, breast cancer, prostate cancer, basal cell carcinoma, biliary tract cancer, bone cancer, brain and central nervous system cancer (e.g., glioma), adenocarcinomas, lung cancer, cervical cancer, choriocarcinoma, colon and rectum cancer, connective tissue cancer, cancer of the digestive system; cancer of the small intestine and cecum; endometrial cancer, esophageal cancer; eye cancer; cancer of the head and neck; gastric cancer; intra-epithelial neoplasm; kidney cancer; larynx cancer; leukemia; liver cancer; gall bladder cancer lung cancer (e.g., small cell and non-small cell); lymphoma including Hodgkin's and Non-Hodgkin's lymphoma; melanoma; myeloma, neuroblastoma, oral cavity cancer (e.g., lip, tongue, mouth, and pharynx); cancer of the salivary glands; ovarian cancer; pancreatic cancer, retinoblastoma; rhabdomyosarcoma; rectal cancer, renal cancer, cancer of the respiratory system; sarcoma, skin cancer; stomach cancer, testicular cancer, thyroid cancer; uterine cancer, cancer of the urinary system, as well as other carcinomas and sarcomas.

In another embodiment of the foregoing: (i) methods of treating cancer, (ii) uses in a method of treating cancer, or (iii) uses in the manufacture of a medicament for treating cancer, the cancer is a cold tumor or a hot tumor.

In a seventh aspect of the present invention, there is provided a kit comprising a costimulatory polypeptide according to the above first aspect or an embodiment thereof, or an oncolytic virus expressing a costimulatory polypeptide according to above second aspect or an embodiment thereof, optionally wherein the kit comprising further reagents.

In an eighth aspect, there is provided the use of an effective amount of costimulatory polypeptide according to the above first aspect or an embodiment thereof, or of an effective amount of oncolytic virus expressing a costimulatory polypeptide according to the above second aspect or an embodiment thereof in an *in vitro* method for stimulating 4-1BB expressing cells.

Further details of the present invention will now be disclosed in detail in the following with additional reference to the attached Drawings, Sequences and the below Examples.

### Brief Description of Drawings

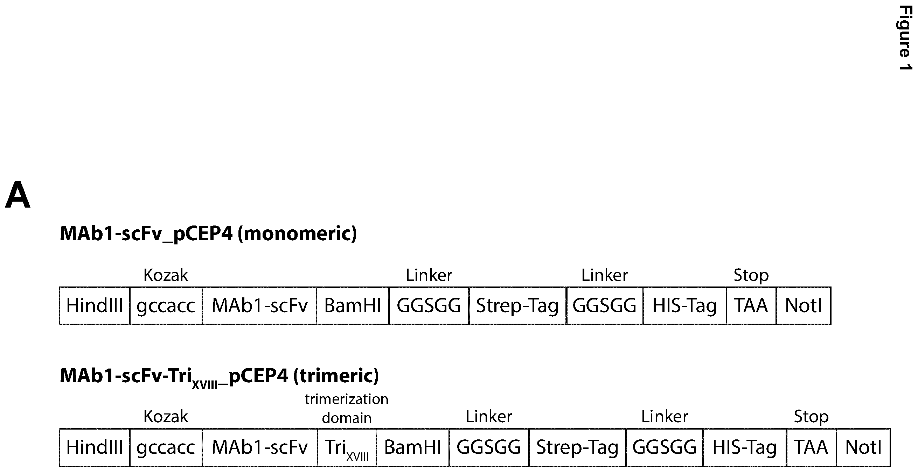

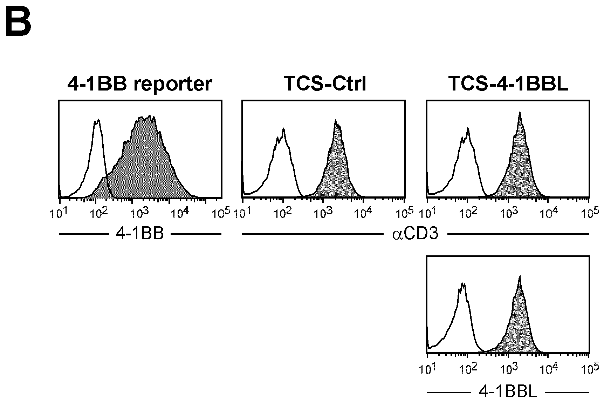

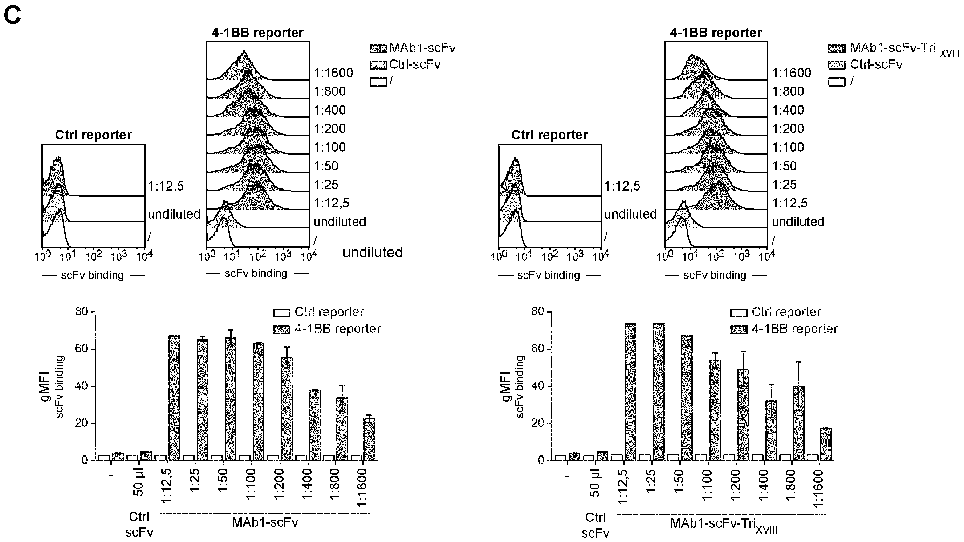

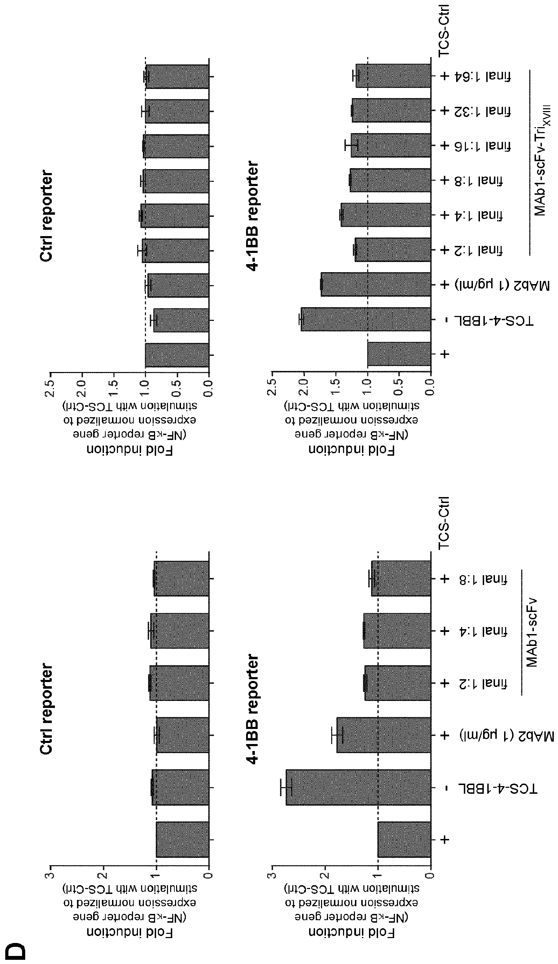

**Figure 1A** shows the graphical representation of the polypeptides MAb1-scFv and MAb1-scFv-Tri_{XVIII}. **Figure 1B** shows the expression levels of 4-1BB on 4-1BB reporter cells as well as the expression levels of anti-CD3 and 4-1BBL on TCS-Ctrl and TCS-4-1 BBL cells (white histograms: control cells not expressing the target molecule, grey histograms: cells that are indicated in respective heading). **Figure 1C** shows the results of the binding assay of the polypeptides MAb1-scFv and MAb1-scFv-Tri_{XVIII} to Ctrl reporter and 4-1 BB reporter cells as histograms or bar charts. **Figure 1D** shows the results of the functional assay of the polypeptides MAb1-scFv and MAb1-scFv-Tri_{XVIII} using Ctrl reporter and 4-1 BB reporter cells. Results are shown as "Fold induction" of NF-κB-dependent eCFP expression, normalized to stimulation with TCS-Ctrl.

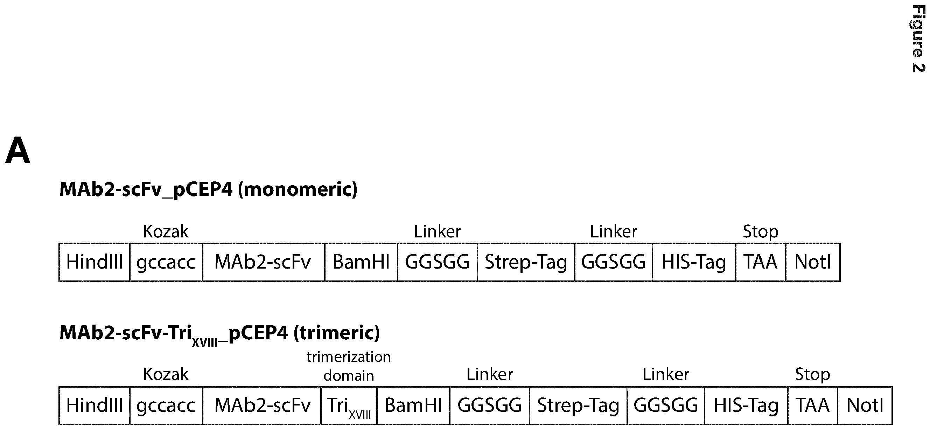

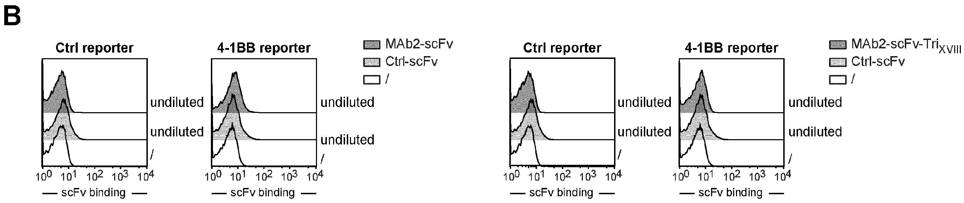

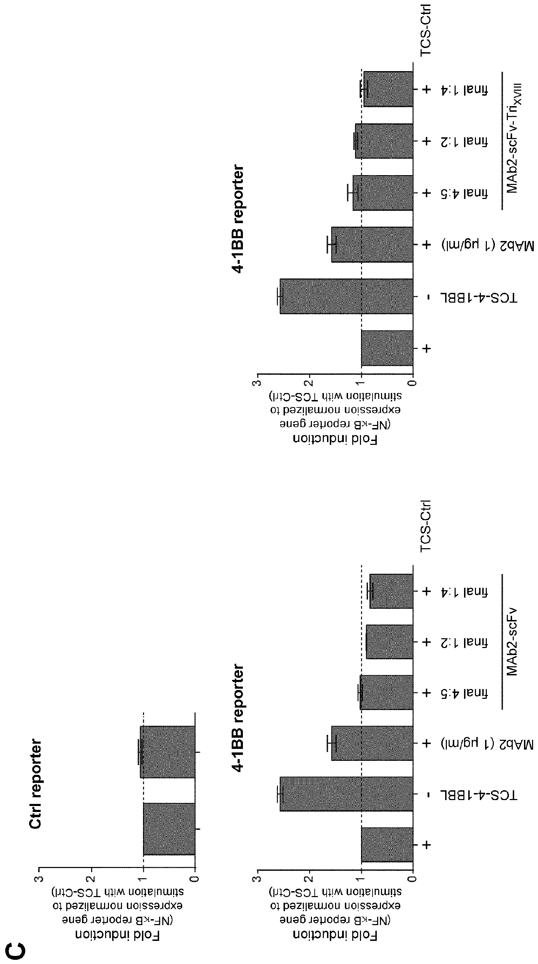

**Figure 2A** shows the graphical representation of the polypeptides MAb2-scFv and MAb2-scFv-Tri_{XVIII}. **Figure 2B** shows the results of the binding assay of the polypeptides MAb2-scFv and MAb2-scFv-Tri_{XVIII} to Ctrl reporter and 4-1 BB reporter cells as histograms. **Figure 2C** shows the results of the functional assay of the polypeptides MAb2-scFv and MAb2-scFv-Tri_{XVIII} using Ctrl reporter and 4-1BB reporter cells. Results are shown as "Fold induction" of NF-κB-dependent eCFP expression, normalized to stimulation with TCS-Ctrl.

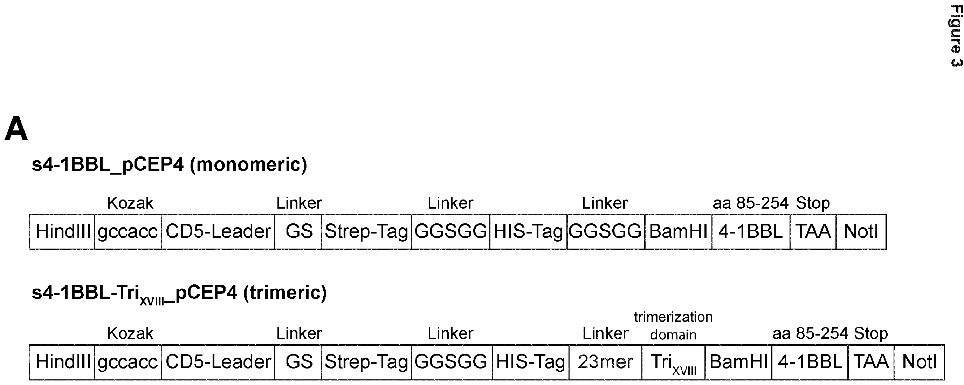

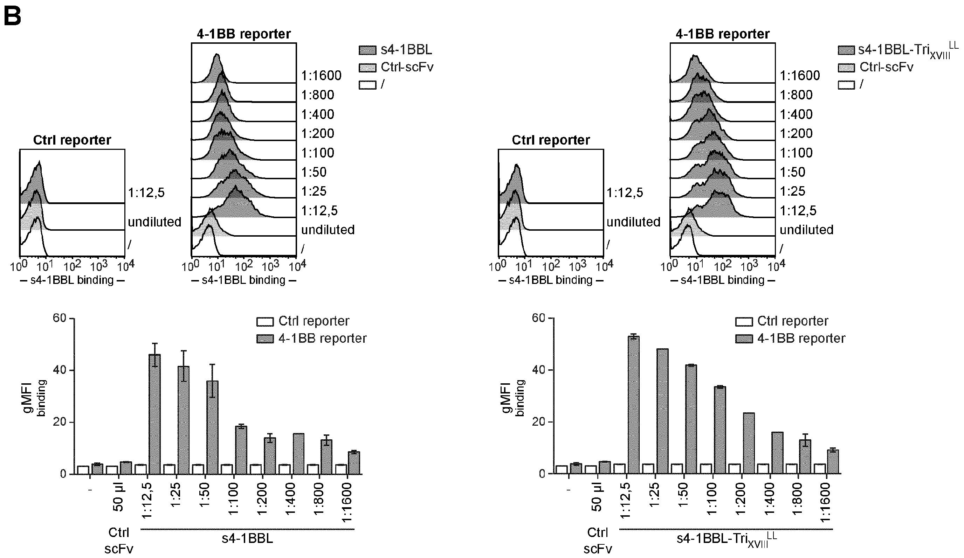

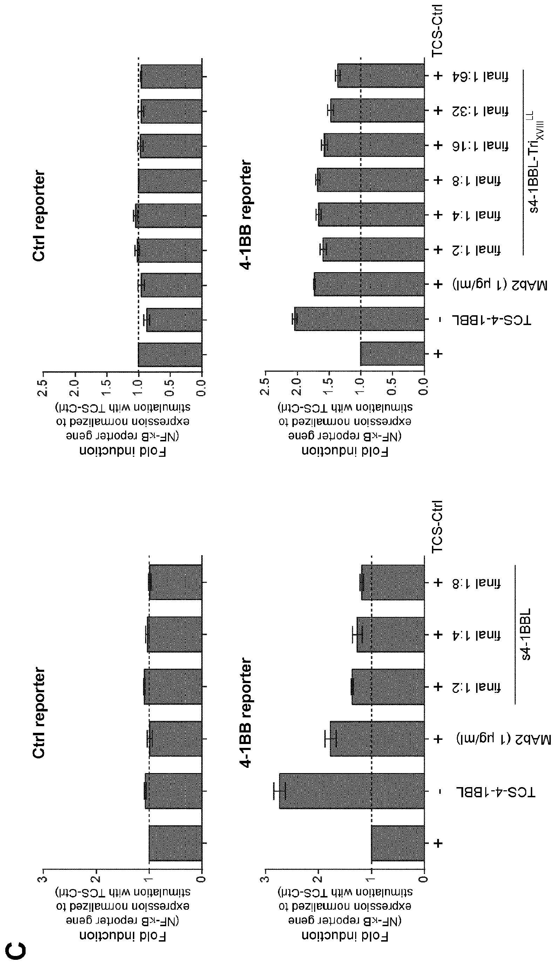

**Figure 3A** shows the graphical representation of the polypeptides s4-1BBL and s4-1BBL-Tri_{XVIII}^{LL}. **Figure 3B** shows the results of the binding assay of the polypeptides s4-1BBL and s4-1BBL-Tri_{XVIII}^{LL} to Ctrl reporter and 4-1BB reporter cells as histograms or bar charts. **Figure 3C** shows the results of the functional assay of the polypeptides s4-1BBL and s4-1BBL-Tri_{XVIII}^{LL} using Ctrl reporter and 4-1BB reporter cells. Results are shown as "Fold induction" of NF-κB-dependent eCFP expression, normalized to stimulation with TCS-Ctrl.

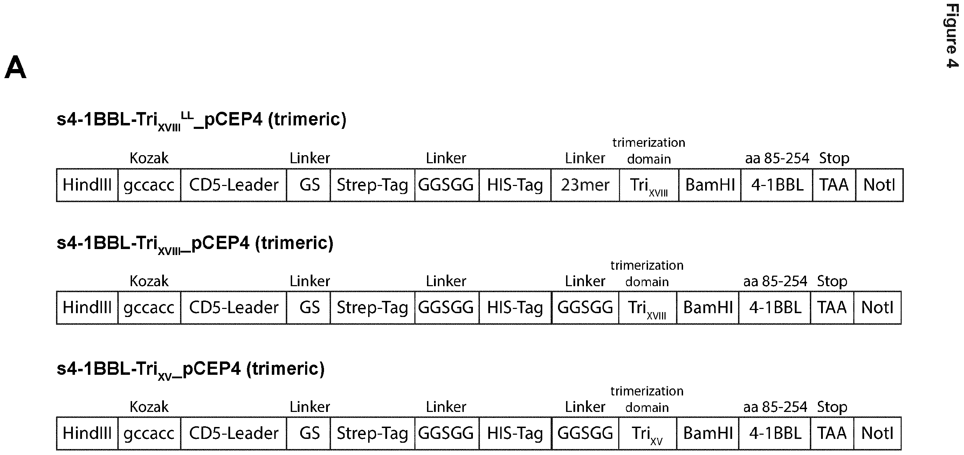

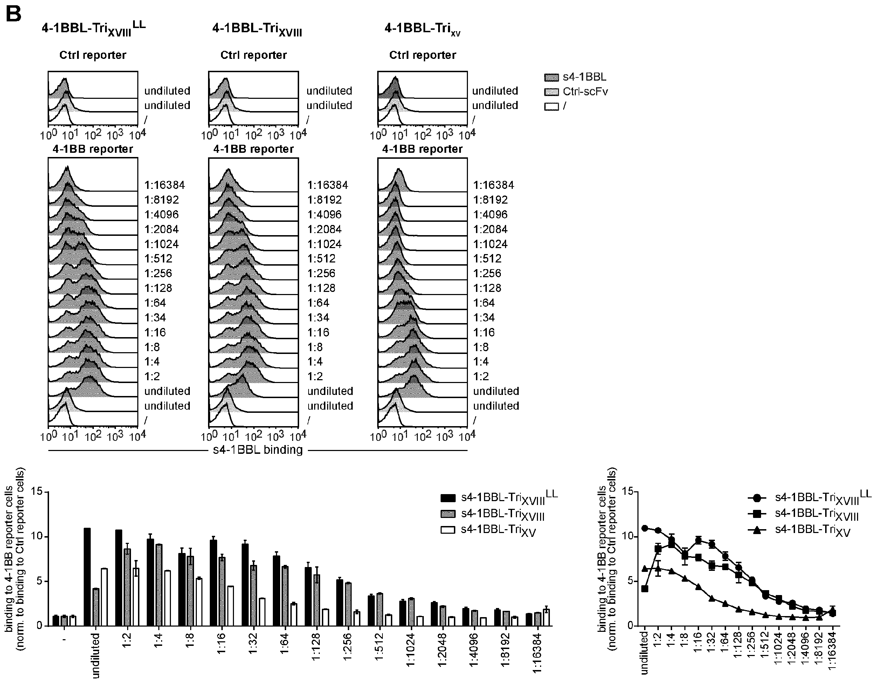

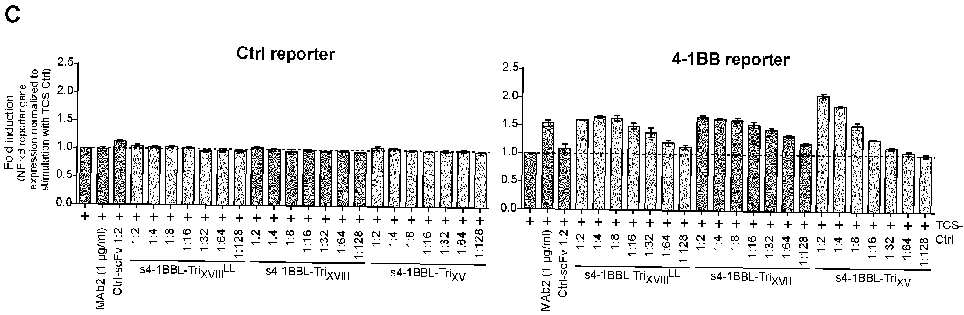

**Figure 4A** shows the graphical representation of the polypeptides s4-1BBL-Tri_{XVIII}^{LL}, s4-1BBL-Tri_{XVIII}, and s4-1 BBL-Trixv. **Figure 4B** shows the results of the binding assay of the polypeptides s4-1BBL-Tri_{XVIII}^{LL}, s4-1BBL-Tri_{XVIII} and s4-1BBL-Tri_{XV}to Ctrl reporter and 4-1BB reporter cells as histograms or bar charts. **Figure 4C** shows the results of the functional assay of the polypeptides s4-1BBL-Tri_{XVIII}^{LL}, s4-1BBL-Tri_{XVIII} and s4-1 BBL-Trixv using Ctrl reporter and 4-1 BB reporter cells. Results are shown as "Fold induction" of NF-κB-dependent eCFP expression, normalized to stimulation with TCS-Ctrl.

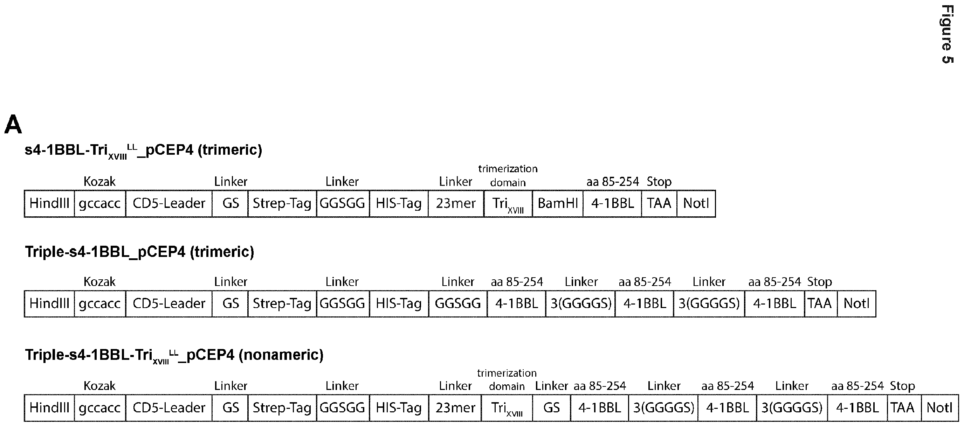

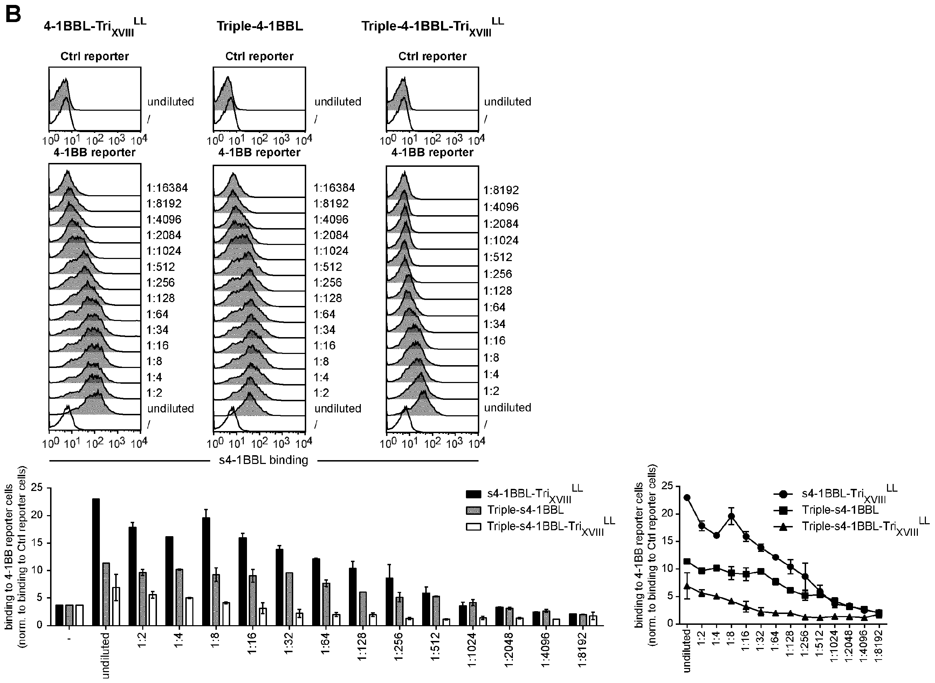

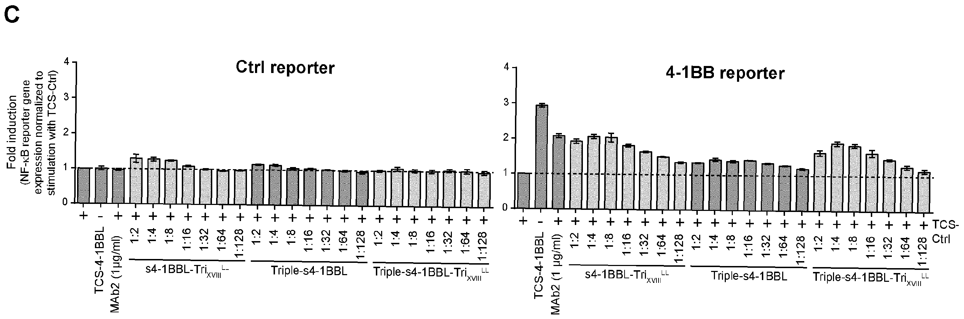

**Figure 5A** shows the graphical representation of the polypeptides s4-1BBL-Tri_{XVIII}^{LL}, Triples4-1BBL and Triple-s4-1BBL- Tri_{XVIII}^{LL}. **Figure 5B** shows the results of the binding assay of the polypeptides s4-1BBL-Tri_{XVIII}^{LL}, Triple-s4-1BBL and Triple-s4-1BBL-Tri_{XVIII}^{LL} to Ctrl reporter and 4-1 BB reporter cells as histograms, bar chart or line plot. **Figure 5C** shows the results of the functional assay of the polypeptides s4-1BBL-Tri_{XVIII}^{LL}, Triple-s4-1BBL and Triple-s4-1BBL-Tri_{XVIII}^{LL} using Ctrl reporter and 4-1 BB reporter cells. Results are shown as "Fold induction" of NF-κB-dependent eCFP expression, normalized to stimulation with TCS-Ctrl.

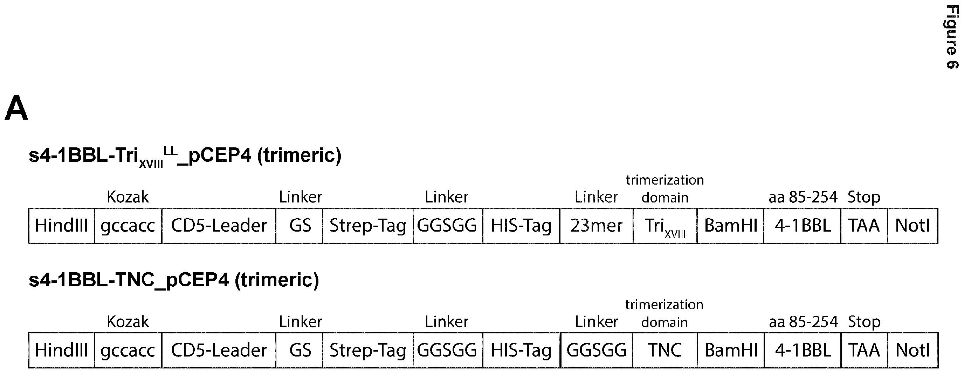

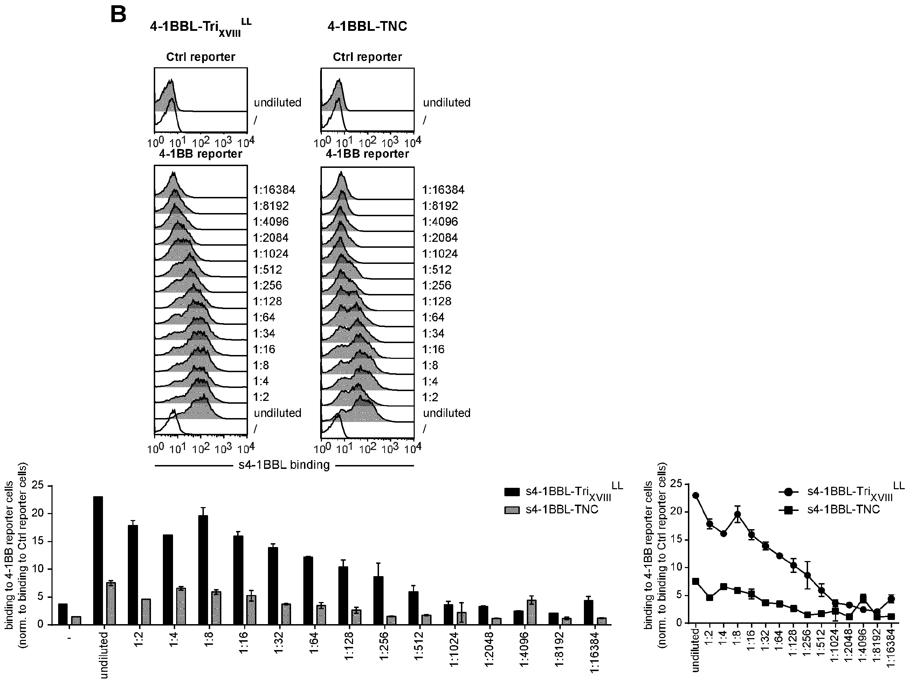

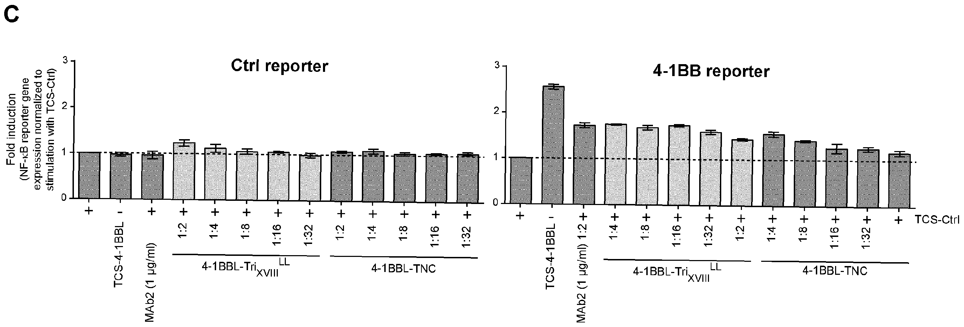

**Figure 6A** shows the graphical representation of the polypeptides s4-1BBL-Tri_{XVIII}^{LL} and s4-1 BBL-TNC. **Figure 6B** shows the results of the binding assay of the polypeptides s4-1BBL-Tri_{XVIII}^{LL} and s4-1 BBL-TNC to Ctrl reporter and 4-1 BB reporter cells as histograms, bar chart or line plot **Figure 6C** shows the results of the functional assay of the polypeptides s4-1BBL-Tri_{XVIII}^{LL} and s4-1 BBL-TNC using Ctrl reporter and 4-1 BB reporter cells. Results are shown as "Fold induction" of NF-κB-dependent eCFP expression, normalized to stimulation with TCS-Ctrl.

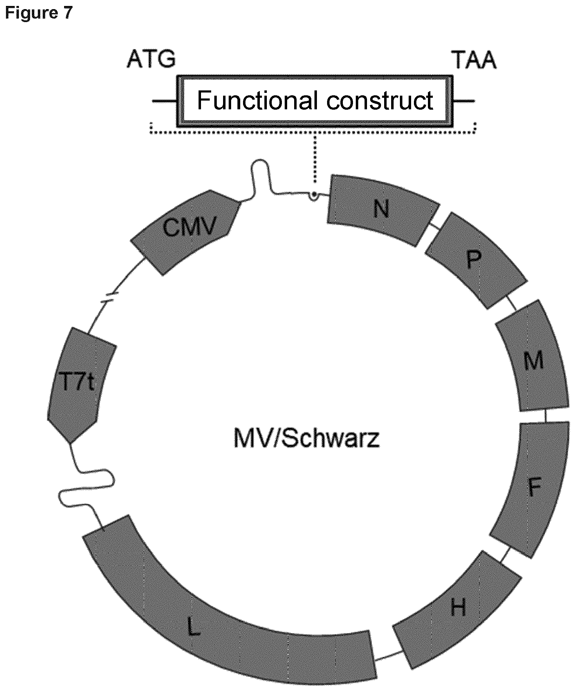

**Figure 7** shows a graphical representation of the possible positioning of a functional polypeptide of the present disclosure in a measles vector of the Schwarz strain.

### Brief Description of Sequences

| SEQ ID NO | Description |
|---|---|
| 1 | Minimal sequence of TNF homology domain (AA 90 to 242 of wild-type 4-1BBL) |
| 2 | Reference sequence of wild-type 4-1 BBL (AA 1 to 254) |
| 3 | 4-1BBL ectodomain polypeptide without cysteine (AA 52 to 254 of wild-type 4-1BBL) |
| 4 | 4-1 BBL ectodomain polypeptide (AA 85 to 254 of wild-type 4-1 BBL) |
| 5 | 4-1 BBL ectodomain polypeptide (AA 52 to 242 of wild-type 4-1 BBL) |
| 6 | 4-1 BBL ectodomain polypeptide (AA 85 to 242 of wild-type 4-1 BBL) |
| 7 | Trimerization domain XVIII |
| 8 | Trimerization domain XV |
| 9 | Linker 1 |
| 10 | Linker 2 (Linker 1 + Tri-Linker) |
| 11 | Linker 3 15mer linker for triple polypeptides |
| 12 | Linker 4 as quadruple sequence, may be used at a variable number of GS dimer |
| 13 | Linker 5 18mer |
| 14 | Linker 6 14mer |
| 15 | Linker 7 |
| 16 | Linker 8 |
| 17 | CD5 leader sequence |
| 18 | Strep tag |
| 19 | Hexa-HIS TAG |
| 20 | s4-1BBL MV(Schwarz)-CMV(1) vector |
| 21 | s4-1BBL in pCEP4 |
| 22 | s4-1BBL in MV(Rubeovax)-CMV(1) vector |
| 23 | s4-1BBL in pAV(adenovirus)-CMV vector |
| 24 | s4-1 BBL-Tri_{XV} in MV(Schwarz)-CMV(1) vector |
| 25 | s4-1 BBL-Tri_{XV} in pCEP4 vector |
| 26 | s4-1 BBL-Tri_{XV} in MV(Rubeovax)-CMV(1) vector |
| 27 | s4-1 BBL-Tri_{XV} in pAV-CMV vector |
| 28 | s4-1BBL-Tri_{XVIII} in MV(Schwarz)-CMV(1) vector |
| 29 | s4-1BBL-Tri_{XVIII}^{LL} in MV(Schwarz)-CMV(1) vector |
| 30 | s4-1 BBL-Tri_{XVIII} in pCEP4 vector |
| 31 | s4-1BBL-Tri_{XVIII} in MV(Rubeovax)-CMV(1) vector |
| 32 | s4-1BBL-Tri_{XVIII}^{LL} in MV(Rubeovax)-CMV(1) vector |
| 33 | s4-1 BBL-Tri_{XVIII} in pAV-CMV vector |
| 34 | Triple s4-1BBL in MV(Schwarz)-CMV(1) vector |
| 35 | s4-1 BBL-Tri_{XVIII}^{LL} in pCEP4 vector |
| 36 | Triple s4-1BBL in pCEP4 vector |
| 37 | Triple s4-1BBL in MV(Rubeovax)-CMV(1) vector |
| | |
| 38 | Triple s4-1BBL in pAV-CMV vector |
| 39 | Triple s4-1BBL-Tri_{XVIII} in MV(Schwarz)-CMV(1) vector |
| 40 | Triple s4-1BBL-Tri_{XVIII}^{LL} in MV(Schwarz)-CMV(1) vector |
| 41 | Triple s4-1BBL- Tri_{XVIII} in pCEP4 vector |
| 42 | Triple s4-1 BBL-Tri_{XVIII}^{LL} in pCEP4 vector |
| 43 | Triple-s4-1 BBL-Tri_{XVIII} in MV(Rubeovax)-CMV(1) vector |
| 44 | Triple-s4-1 BBL-Tri_{XVIII}^{LL} in MV(Rubeovax)-CMV(1) vector |
| 45 | Triple-s4-1 BBL-Tri_{XVIII} in pAV-CMV vector |
| 46 | Triple-s4-1 BBL-Tri_{XVIII}^{LL} in pAV-CMV vector |
| 47 | s4-1 BBL; without tags (DNA) |
| 48 | s4-1BBL; without tags; protein (PROT) |
| 49 | s4-1 BBL; with tags (DNA) |
| 50 | s4-1 BBL; with tags (PROT) |
| 51 | s4-1 BBL-Tri_{XV}; without tags (DNA) |
| 52 | s4-1 BBL-Tri_{XV}; without tags (PROT) |
| 53 | s4-1 BBL-Tri_{XV}; with tags (DNA) |
| 54 | s4-1 BBL-Tri_{XV}; with tags (PROT) |
| 55 | s4-1 BBL-Tri_{XVIII}; with tags (DNA) |
| 56 | s4-1 BBL-Tri_{XVIII}; with tags (PROT) |
| 57 | s4-1 BBL-Tri_{XVIII}; without tags (DNA) |
| 58 | s4-1 BBL-Tri_{XVIII}; without tags (PROT) |
| 59 | s4-1 BBL-Tri_{XVIII}^{LL}; without tags (DNA) |
| 60 | s4-1 BBL-Tri_{XVIII}^{LL}; without tags (PROT) |
| 61 | Triple-s4-1 BBL; without tags (DNA) |
| 62 | Triple-s4-1 BBL; without tags (PROT) |
| 63 | Triple-s4-1 BBL; with tags (DNA) |
| 64 | Triple-s4-1 BBL; with tags (PROT) |
| 65 | Triple-s4-1 BBL-Tri_{XVIII}; without tags (DNA) |
| 66 | Triple-s4-1 BBL-Tri_{XVIII}; without tags (PROT) |
| 67 | Triple-s4-1 BBL-Tri_{XVIII}^{LL}; without tags (DNA) |
| 68 | Triple-s4-1 BBL-Tri_{XVIII}^{LL}; without tags (PROT) |
| 69 | Triple-s4-1 BBL-Tri_{XVIII}^{LL}; with tags (DNA) |
| 70 | Triple-s4-1 BBL-Tri_{XVIII}^{LL}; with tags (PROT) |
| 71 | Triple-s4-1 BBL-Tri_{XVIII}; with tags (DNA) |
| 72 | s4-1 BBL-Tri_{XVIII}^{LL}; with tags; with Linker 8 (PROT) |
| 73 | s4-1 BBL-Tri_{XVIII}^{LL}; with tags; with Linker 8 (DNA) |
| 74 | Triple-s4-1 BBL-Tri_{XVIII}^{LL}; with tags; with Linker 7 (PROT) |
| 75 | Triple-s4-1 BBL-Tri_{XVIII}^{LL}; with tags; with Linker 7 (DNA) |
| 76 | s4-1 BBLsh-Tri_{XVIII}; with tags (PROT) |
| | |
| 77 | s4-1 BBLsh-Tri_{XVIII}; with tags (DNA) |
| 78 | Triple-s4-1 BBL-Tri_{XVIII}; with tags; with Linker 1 (PROT) |
| 79 | Triple-s4-1 BBL-Tri_{XVIII}; with tags; with Linker 1 (DNA) |
| 80 | Kozak sequence (n can be any base and depicts the generic gene downstream of the prototypic Kozak sequence) |
| 81 | Hammerhead ribozyme sequence |
| 82 | s4-1BBL TNC in MV(Schwarz)-CMV(1) vector |
| 83 | s4-1BBL TNC in pCEP4 vector |
| 84 | s4-1BBL-TNC in MV(Rubeovax)-CMV(1) vector |
| 85 | s4-1 BBL-TNC in pAV-CMV vector |
| 86 | s4-1 BBL-TNC with tags (PROT) |
| 87 | MAb1 scFv without tags (PROT) |
| 88 | MAb1-scFv; without tags (DNA) |
| 89 | MAb2-scFv-Tri_{XVIII}; without tags (PROT) |
| 90 | MAb2-scFv-Tri_{XVIII}; without tags(DNA) |
| 91 | Signal peptide derived from the human Igκ light chain |
| 92 | Complementarity-defining region (CDR) VH of MAb1 |
| 93 | CDR VL region of MAb1 |
| 94 | Primer MAb1-F-Hindlll |
| 95 | Primer MAb1-B1 |
| 96 | Primer B2-Notl |
| 97 | Primer MAb1-B |
| 98 | Primer MAb1-Tri-F |
| 99 | Primer TriDom-B-BamHI |
| 100 | CDR VH of MAb2 |
| 101 | CDR VL region of MAb2 |
| 102 | Primer Tag-4-1 BBL-F1 |
| 103 | Primer 4-1 BB-L-B-NotI |
| 104 | Primer CD5L-Tags-F2-Hindlll |
| 105 | Primer Tags-TriDom-F1 |
| 106 | Primer TriDom-4-1 BB-L-B |
| 107 | Primer TriDom-4-1 BB-L-F |
| 108 | Primer Triple-s4-1 BBL-F-Hindlll |
| 109 | Primer Triple-s4-1 BBL-B-NotI |
| 110 | Chicken tenascin-C (AA 110 to 139) |
| 111 | Primer Tags-TNC-B |
| 112 | Primer Tags-TNC-F |
| 113 | Primer MAb2-F-Hindlll |
| 114 | Primer MAb2-B-BamHI |
| 115 | s4-1 BBL-Tri_{XVIII}^{LL} pAV-CMV |
| 116 | s4-1 BBLsh (short) -Tri_{XVIII} in MV(Schwarz)-CMV(1) vector |
| 117 | s4-1 BBLsh-Tri_{XVIII} in pCEP4 vector |
| 118 | s4-1 BBLsh-Tri_{XVIII} in MV(Rubeovax)-CMV(1) vector |
| 119 | s4-1 BBLsh-Tri_{XVIII} in pAV-CMV vector |

### Definitions

Unless defined otherwise, technical and scientific terms used herein have the same meaning as commonly understood by one of ordinary skill in the art to which this invention belongs.

As used in the context of the present application, the term "about" means +/- 10% of the recited value, preferably +/- 5% of the recited value. For example, about 100 nucleotides (nt) shall be understood as a value between 90 and 110 nt, preferably between 95 and 105 nt.

An "antibody" or "immunoglobulin" (Ig) as used herein refers to a polypeptide molecule including naturally occurring immunoglobulins, monoclonal antibodies including murine, human, humanized and chimeric monoclonal antibodies, antibody fragments, bispecific or multispecific antibodies, dimeric, tetrameric or multimeric antibodies, single chain antibodies, single domain antibodies, antibody mimetics and any other modified configuration of the immunoglobulin molecule that comprises an antigen binding site of the required specificity. An "antibody" usually comprises two heavy chains and two light chains connected by disulphide bonds, as well as any multimer thereof (e.g. IgM). Each heavy chain comprises a heavy chain variable region (VH) and a heavy chain constant region (comprising the domains CH1, hinge, CH2 and CH3 as well as CH4 for IgM and IgE). Each light chain usually comprises of a light chain variable region (VL) and a light chain constant region (CL). The VH and the VL regions may be further subdivided into regions of hypervariability, termed "complementarity determining regions" (CDR), interspersed with framework regions (FR). These CDRs represent the actual target or antigen-binding site of an antibody. Each VH and VL is composed of three CDRs and four FR segments, arranged from amino-to-carboxyl-terminus in the following order: FR1, CDR1, FR2, CDR2, FR3, CDR3 and FR4. Immunoglobulins are usually assigned to five major classes, namely IgA, IgD, IgE, IgG and IgM, wherein the categorization depends on the heavy chain constant region architecture of an immunoglobulin. IgA and IgG are further sub-classified as the isotypes IgA1, IgA2, IgG1, IgG2, IgG3 and IgG4. Regarding the light chains of an antibody or immunoglobulin, there are usually two types, namely kappa (K) and lambda (I), again based on the architecture of the constant regions.

An "aptamer" as used herein refers to an oligonucleotide (RNA and/or DNA) or peptide molecule that binds to a specific target molecule. Aptamers can include naturally occurring or synthetic nucleotides and/or amino acids, or at least one nucleotide or amino acid position, or the linkage between two nucleotides or amino acids, may be synthetically modified, for example, a nucleic acid analogues.

A "checkpoint inhibitor" is a biological molecule or a chemical substance that blocks proteins called "checkpoints" or "immune checkpoints" (ICPs) that are expressed by some types of immune system cells, such as T-cells, and particularly by some cancer cells. These checkpoints help keep immune responses from being too strong and sometimes can keep T-cells from killing cancer cells. When these checkpoints are blocked, T-cells can more effectively kill cancer cells. Examples of checkpoint proteins found on T cells or cancer cells include, for example, PD-1, PD-L1, and CTLA-4. "Immune checkpoint inhibitors" may thus be used, usually together with other compositions or compounds, to treat cancer.

The term "cold tumor" or "non-inflamed tumor" as used herein refers to tumor or tumor tissue generally characterized by a lack of infiltrating T-cells. This lack of infiltrating T-cells can be due to the lack of tumor antigens, APC deficits, or the absence of T cell priming/activation and impaired trafficking of T-cells to the tumor mass (cf. Bonaventura et al., Frontiers in Immunology, vol. 10, 2019). In contrast, a "hot tumor" is usually characterized by a "T-cell inflamed" phenotype. Cancer immunotherapy aims at breaking the impaired T-cell infiltration in cold tumors to make the tumor tissue accessible for T-cells of the immune system.

A "genome" as used herein is to be understood broadly and comprises any kind of genetic information (RNA/DNA) inside any compartment of a living cell. In the context of a "genome modification", the term thus also includes artificially introduced genetic material, which may be transcribed and/or translated, inside a living cell, for example, an episomal plasmid or vector, or an artificial DNA integrated into a naturally occurring genome.

The term "costimulatory" in context of the present invention means that certain immune cells, such as B-lymphocytes and T-lymphocytes require a second signal to be fully activated. This is for example the case for naive CD8⁺ T-lymphocytes. The primary stimulus is the binding of the MHC class I on the surface of antigen-presenting cells to the T-cell receptor. Without a second signal, the cell is not being optimally activated. A second stimulus is required, such as the binding of 4-1BB on the surface of antigen-presenting cells to 4-1BBL on the surface of activated T-cells. After full activation by binding of 4-1BB to 4-1BBL, T-cell proliferation, IL-2 secretion and cytolytic activity is enhanced.

A "linker" in terms of the present invention is a sequence of a polypeptide, which separates two functional domains of a polypeptide spatially by forming a defined secondary structure allowing flexibility and/or spacing of the domains separated and thus full functionality if the domains such separated. The general properties of linkers are usually classified into three categories according to their structures: flexible linkers, rigid linkers, and *in vivo* cleavable linkers (Chen et al., Adv. Drug. Deliv. Rev., 2013, 65(10), 1357-1369). All of these linkers may be used according to the disclosure presented herein, alone, or in combination. Typical linker sequences can form different secondary structures such as loops, helices and linear structures. A linker sequence can be present anywhere in a multidomain polypeptide, preferably to separate individual functional domain. Linker sequences can be especially present between subunits of a protein, or between two domains of a multidomain protein, or between a functional element and a protein element before or after tag sequences. It is well known in the art, that different linkers can be present and how a linker has to be designed to fulfill its purpose. Several linkers and the use thereof in the polypeptides of the present invention are provided below. Further linkers are available to the skilled person and can be used according to the present disclosure.

The term "oncolytic virus" or "OV" as used herein refers to a naturally occurring or an engineered virus, which can be used in cancer therapy. An OV usually has engineered and/or intrinsic tropism for cancer cells, e.g. by using cancer-cell proteins as receptor, and/or possesses preferential replication in cancer cells. Besides, causing the death of virus-infected cancer cells, the intratumoral (IT) infection can also boost the anticancer immune response, leading to immune destruction of uninfected cancer cells. OVs may be engineered to comprise sequences encoding additional "payloads". These "payloads" are molecules expressed by the OV that additionally favor an anti-tumor response, by co-stimulation of T-cells, or by reducing undesired responses, or by breaking checkpoints etc. An OV may also be a chimera, i.e. the OV is encoded by a nucleic acid molecule of at least two different biological origins, and/or the OV combines naturally occurring and artificial elements. A "chimeric virus" or "chimera" OV may thus refer to at least one virus particle originating from at least two nucleic acid molecules of different organisms/viruses, or from a recombinant (artificial) molecule. Further, virus particles, including OVs, may also optionally comprise host cell derived material (e.g. a host cell derived lipid bilayer), which the virus incorporates during processing and particle formation within the host cell after replication of its genome.

The term "operatively linked/connected", "operably linked/connected", or "functionally linked/connected" specifically in the context of nucleic acids, for example plasmids or expression vectors, or an OV as a vector, means that one element, for example, a regulatory element, or a first protein-encoding sequence, is linked in such a way with a further part so that the protein-encoding nucleotide sequence, i.e., is positioned in such a way relative to the protein-encoding nucleotide sequence on, for example, a nucleic acid molecule that an expression of the protein-encoding nucleotide sequence under the control of the regulatory element can take place in a living cell.

The term "payload" in terms of the present invention means the delivery of accessory sequences to be expressed in a tumor cell by an oncolytic virus. These sequences encode immune stimulatory, cytotoxic or replication blocking agents, which act as an additional effector molecule besides the virus induced lysis of tumor cells.

The terms "protein" and "polypeptide" are used interchangeably herein for amino acid sequences encoding a functional protein or enzyme, or a functional fragment thereof. The term "peptide" as used herein refers to shorter polypeptide sequences of usually below about 100 amino acids in length.

As used herein, a "regulatory sequence", or "regulatory element" refers to nucleotide sequences which are not part of an RNA/protein-encoding nucleotide sequence, but which mediate the expression of the RNA/protein-encoding nucleotide sequence. Regulatory elements include, for example, promoters, cis-regulatory elements, enhancers, introns or terminators. Depending on the type of regulatory element it is located on the nucleic acid molecule before (i.e., 5' of) or after (i.e., 3' of) the protein-encoding nucleotide sequence. Regulatory elements are functional in a living cell.

A "single-chain antibody" (scAb) or "single-chain Fv fragment" (scFv) as used herein refers to a polypeptide molecule modified by means of genetic engineering, which contains the variable light chain region and the variable heavy chain region of an antibody molecule, usually connected by a suitable peptide linker.

The term "tag" or "protein tag" refers to a variety of different polypeptide sequences or the corresponding nucleic acid sequences encoding the same, which can be incorporated into a protein of interest at various positions, preferably at the N- and/or C-terminus, or between distinct functional domains, and fulfil several different functions. Protein tags can, for example, be differentiated into affinity tags such as chitin binding protein (CBP), maltose binding protein (MBP), Strep-tag and glutathione-S-transferase (GST) or His-Tag. Affinity tags can be used for specifically purifying the protein of interest. Another example of protein tags are solubilization tags, which allow to obtain the protein of interest in its soluble form especially in bacterial cell culture. Epitope tags can be used for having a binding site for analytically antibodies and fluorescence tags offer the possibility to detect the protein of interest in the cell culture.

The term "treat" or "treatment" means to administer a therapeutic agent, such as a composition containing any of the polypeptides or oncolytic viruses of the present invention, internally or externally to a subject or patient having one or more disease symptoms, or being suspected of having a disease, for which the agent has therapeutic activity. Typically, the agent is administered in an amount effective to alleviate one or more disease symptoms in the treated subject or population, whether by inducing the regression of or inhibiting, delaying or slowing the progression of such symptom(s) by any clinically measurable degree. The amount of a therapeutic agent that is effective to alleviate any particular disease symptom may vary according to factors such as the disease state, age, and weight of the patient, and the ability of the drug to elicit a desired response in the subject. Whether a disease symptom has been alleviated can be assessed by any clinical measurement typically used by physicians or other skilled healthcare providers to assess the severity or progression status of that symptom. The term further includes a postponement of development of the symptoms associated with a disorder and/or a reduction in the severity of the symptoms of such disorder. The terms further include ameliorating existing uncontrolled or unwanted symptoms, preventing additional symptoms, and ameliorating or preventing the underlying causes of such symptoms. Thus, the terms denote that a beneficial result has been conferred on a vertebrate subject with a disorder, disease or symptom, or with the potential to develop such a disorder, disease or symptom.

The term "trimerizable" in context of the present invention means the ability of a molecule to form a complex structure of three sub structures. More specific, in the field of polypeptides, the term trimerizable means the ability of a polypeptide monomer to form a structure complex together with two similar or identical other monomers resulting in a trimer.

As used herein, "upstream" indicates a location on a nucleic acid molecule, which is nearer to the 5' end of said nucleic acid molecule. Likewise, the term "downstream" refers to a location on a nucleic acid molecule which is nearer to the 3' end of said nucleic acid molecule. For avoidance of doubt, nucleic acid molecules and their sequences are typically represented in their 5' to 3' direction (left to right).

The terms "vector", or "plasmid (vector)" refer to a molecule that can replicate in a cell independently from a chromosome, including but not limited to plasmids or (plasmid) vectors, viruses, cosmids, artificial yeast- or bacterial artificial chromosomes (YACs and BACs), phagemids, and bacterial phage-based vectors. Vectors may comprise, *inter alia,* an expression cassette, isolated single-stranded or double-stranded nucleic acid sequences, comprising sequences in linear or circular form that encode amino acid sequences, viruses, viral replicons including modified viruses, and combinations or s mixtures thereof, for introduction or transformation, transfection or transduction into any eukaryotic cell, including any kind of eukaryotic cell, tissue, organ or material according to the present disclosure. A "nucleic acid vector", for instance, is a DNA or RNA molecule, which is used to deliver foreign genetic material to a cell, where it can be transcribed and optionally translated, such as a plasmid or expression vector. Preferably, the vector is a plasmid comprising multiple cloning sites. The vector may further comprise a "unique cloning site" a cloning site that occurs only once in the vector and allows insertion of DNA sequences, e.g. a nucleic acid cassette or components thereof, by use of specific restriction enzymes. A "flexible insertion site" may be a multiple cloning site, which allows insertion of the components of the nucleic acid cassette according to the invention in an arrangement, which facilitates simultaneous transcription of the components and allows activation of the RNA activation unit. An "Additional Transcription Unit" or "ATU" is an example for a flexible insertion site. An ATU may usually comprise three potential regions of inserting a nucleic acid and further comprise, for use in steps of cloning into cDNA of MV, cis-acting sequences necessary for MV-dependent expression of a recombinant transgene, such as a promoter preceding a gene of interest, into the nucleic acid molecule encoding an oncolytic virus, for example, a measles virus (MV). The ATU may serve the function of a multiple cloning site. More than one ATU may be present. An ATU must guarantee that the normal replicative functions of an oncolytic virus of interest are not interrupted. For MV, an ATU may be located in the N-terminal sequence of the cDNA molecule encoding the full-length (+)RNA strand of the antigenome of the MV, for example before the N gene (ATU1), and, for the purpose of the present disclosure, it is preferably located between the P and M genes of this virus (ATU2). Alternatively, it can be located between the H and L genes (ATU3). The term "rescue" describes procedures that are necessary to construct and isolate recombinant viruses, which have an altered genome compared to the original virus strain. These procedures can be very different for different virus types and are known to persons skilled in the art.

Whenever the present disclosure relates to the percentage of the homology or identity of nucleic acid or amino acid sequences, this identity implies a comparison over the entire length of the respective sequence to be compared to another, wherein the sequence of interest or subject represents the reference sequence (e.g., in the form of a SEQ ID NO as disclosed herein). These identity or homology values define those as obtained by using the EMBOSS Water Pairwise Sequence Alignments (nucleotide) programme (www.ebi.ac.uk/Tools/psa/emboss_water/nucleotide.html) nucleic acids or the EMBOSS Water Pairwise Sequence Alignments (protein) programme (www.ebi.ac.uk/Tools/psa/emboss_waterl) for amino acid sequences. Those tools provided by the European Molecular Biology Laboratory (EMBL) European Bioinformatics Institute (EBI) for local sequence alignments use a modified Smith-Waterman algorithm (see www.ebi.ac.uk/Tools/psa/ and Smith, T.F. & Waterman, M.S. "Identification of common molecular subsequences" Journal of Molecular Biology, 1981 147 (1):195-197). When conducting an alignment, the default parameters defined by the EMBL-EBI are used. Those parameters are (i) for amino acid sequences: Matrix = BLOSUM62, gap open penalty = 10 and gap extend penalty = 0.5 or (ii) for nucleic acid sequences: Matrix = DNAfull, gap open penalty = 10 and gap extend penalty = 0.5.

### Detailed Description

4-1BB is a highly important target for new forms of immuno-stimulatory therapy. Nevertheless, working with polypeptides as reported in the state of the art, such as 4-1 BB targeting scFvs and agonistic antibodies - presently representing the standard agents to modulate 4-1BB signaling - have been reported to result in side effects and undesired systemic immune system activation. Additionally, several artificial polypeptides for 4-1BB targeting and signaling modulation have been created, still these polypeptides may be of particular interest for experimental settings only, as the components of non-human origin may hamper therapeutic applications in a patient.

So far, no attempts have been made to specifically work with artificial polypeptides mimicking the fully natural ligand 4-1BBL of 4-1BB, which represents an immunologically favorable strategy as no sequences recognized as "foreign" by the human immune system have to be used.

To address the needs to provide more specific and active 4-1BB modulating agents, the present invention thus provides in a first aspect a costimulatory polypeptide, wherein the polypeptide may comprise (a) (i) at least one 4-1BBL ectodomain and a trimerization domain, and/or (ii) at least one 4-1BBL ectodomain and at least one leader sequence, and optionally at least one affinity tag; wherein each 4-1BBL ectodomain comprises or consists of the minimal TNF Homology Domain according to SEQ ID NO: 1, or a sequence having at least 90%, 91%, 92%, 93%, 94%, 95%, 96%, 97%, 98% or 99% sequence identity to SEQ ID NO: 1 or a nucleic acid sequence encoding the polypeptide; and/or (b) at least three 4-1BBL ectodomains, and optionally comprising at least one trimerization domain, wherein each 4-1BBL ectodomain comprises or consists of the minimal TNF Homology Domain according to SEQ ID NO: 1, or a sequence having at least 90%, 91%, 92%, 93%, 94%, 95%, 96%, 97%, 98% or 99% sequence identity to SEQ ID NO: 1, or a nucleic acid sequence encoding the polypeptide ; and wherein the 4-1BBL ectodomain does not comprise the cysteine residue at position 51 of the naturally occurring human 4-1BBL ectodomain as shown in SEQ ID NO: 2, or a nucleic acid sequence encoding the same; and wherein the polypeptide binds to 4-1BB on the surface of a 4-1BB expressing cell and thus triggers 4-1BB-mediated immune cell stimulation. In specific embodiments, the trimerization domain of any of the preceding costimulatory polypeptides is selected from SEQ ID NO: 7 or 8.

According to the various aspects of the present invention, a trimerization domain other than SEQ ID NO: 7 or 8, preferably a human or a humanized trimerization domain, may be used in the context of the 4-1BBL costimulatory polypeptides of the present invention.

As detailed above, trimerization is key for obtaining a functional 4-1BBL, as this central ligand functionally interacts with the trimeric 4-1BB in the trimeric state to mediate and initiate downstream signaling. The secretion and assembly of trimeric 4-1BBL ectodomain polypeptides (costimulatory polypeptides) of the present invention results in a superior functionality compared to the monomer secretion and, therefore, a higher order structure properly mimicking the endogenous 4-1BBL can be obtained with the soluble 4-1BBL ectodomain polypeptides of the present invention. Furthermore, an oncolytic virus expressing a secreted and still highly functional s4-1BBL could be designed, which paves the way to use the signaling properties for oncolytic virus therapy in targeted immunomodulation.

The polypeptides of the present invention have several functional domains, which are described in detail below:
Working with a minimal polypeptide of 4-1BBL, i.e., the s4-1BBL costimulatory polypeptide according to SEQ ID NO: 1 as disclosed herein, has several advantages over the polypeptides presently available. Full length natural 4-1BBL comprises a membrane anchor. When designing therapeutics based on the natural ligand, a membrane anchor may have several disadvantages. On the one hand, high expression rates may be impaired, and on the other hand T-cells may mainly be activated by cells that are lysed anyway when the ligand is encoded by OV. Finally, the s4-1BBL as used therapeutically will be fully active without the need of any domain or sequence in the polypeptide being of non-human origin and thus being a potential target of an undesired immune response.

Just recently, new structural insights of the 4-1BB/4-1BBL complex were elucidated showing the distinct binding and functional properties of this complex (Chin et al., Nature Communications, 2018, 9:4679).

In contrast to earlier structural studies, Chin et al. *supra* elucidated markedly different data on the structure of the naturally occurring 4-1BBL homotrimer. This information was used to design various minimal polypeptides using core elements of the 4-1BBL, but always excluding a mutation or truncation in those regions shown to be conserved in receptor ligand interaction. These relevant positions include Q98, V100, Y110, G114, L115, A116, R150, R151, V152, V153, A154, Q227 and Q230 of SEQ ID NO: 2 (see Figure 2C of Chin et al *supra*)*.* Despite these important residues, the polypeptides seemed to tolerate certain further mutations, in particular when substituting an amino acid against an amino acid having the same property, whereas sterically demanding and/or cysteine insertions were avoided (i.e., in the cluster hydrophobic-aliphatic (Ala, Ile, Leu, Met, Val), hydrophobic aromatic (Phe, Trp, Tyr), polar neutral (Asn, Cys, Gln, Ser, Thr), charged acidic (Asp, Glu), charges basic or nearly neutral (Arg, Lys, His), Pro, or nonpolar (Gly) (data not shown).

Further, a true minimal domain of the TNF homology domain (SEQ ID NO: 1) was constructed having maximum flexibility and being devoid of the cysteine residues of the naturally occurring 4-1BBL. Therefore, a core soluble ectodomain s4-1BBL and several variants thereof were constructed maintaining the essential trimerization potential, either intrinsically, or when combined with a trimerization domain and the core TNF homology domain relevant for receptor interaction, but lacking the membrane anchor domain and further amino acid positions hampering a proper and flexible polypeptide design for therapeutic purposes.

In certain embodiments of the various aspects of the present invention, the 4-1BBL ectodomain of the costimulatory polypeptide may thus comprise or consist of a sequence according to SEQ ID NOs: 3 to 6, or a polypeptide with a sequence having at least 90%, 91%, 92%, 93%, 94%, 95%, 96%, 97%, 98% or 99% sequence identity to the respective sequence, or a nucleic acid sequence encoding the same. In specific embodiments, the at least one 4-1BBL ectodomain of the costimulatory polypeptide comprises SEQ ID NO: 3, or a polypeptide with a sequence having at least 90%, 91%, 92%, 93%, 94%, 95%, 96%, 97%, 98% or 99% sequence identity to SEQ ID NO: 3, or a nucleic acid sequence encoding the same. In specific embodiments, the at least one 4-1BBL ectodomain of the costimulatory polypeptide comprises SEQ ID NO: 4, or a polypeptide with a sequence having at least 90%, 91%, 92%, 93%, 94%, 95%, 96%, 97%, 98% or 99% sequence identity to SEQ ID NO: 4, or a nucleic acid sequence encoding the same. In specific embodiments, the at least one 4-1BBL ectodomain of the costimulatory polypeptide comprises SEQ ID NO: 5, or a polypeptide with a sequence having at least 90%, 91%, 92%, 93%, 94%, 95%, 96%, 97%, 98% or 99% sequence identity to SEQ ID NO: 5, or a nucleic acid sequence encoding the same. In specific embodiments, the at least one 4-1BBL ectodomain of the costimulatory polypeptide comprises SEQ ID NO:6, or a polypeptide with a sequence having at least 90%, 91%, 92%, 93%, 94%, 95%, 96%, 97%, 98% or 99% sequence identity to SEQ ID NO: 6, or a nucleic acid sequence encoding the same.

In certain embodiments of the various aspects of the present invention, the at least one 4-1BBL ectodomain polypeptide may comprise at least one trimerization domain. The at least one trimerization domain allows either trimerization as such, or the formation of higher order oligomers (hexamers etc.), which may additionally favor immunomodulation and activity. Such an oligomerization was observed for certain polypeptides of the present invention as favorable effect. In some embodiments, an additional linker between the at least one trimerization domain and the at least one (first) s4-1BBL domain may be used, in other embodiments, the at least one trimerization domain may be directly connected and operably linked to the (first) s4-1BBL domain. Likewise, in some embodiments, an additional linker between the leader sequence and a subsequent domain (tag or trimerization, or s4-1BBL domain) may be used, in other embodiments, this linker may be omitted. Long linkers (abbreviated as "LL"), and short linkers (abbreviated as "sh") and various combinations of these have been tested in connection with the various costimulatory polypeptide of the present invention, either for the recombinant polypeptides as such, or for the polypeptides as expressed in various OVs of different origin. Exemplary polypeptides and nucleic acids encoding these polypeptides are shown in the sequence listing. A trimerization domain may be preferably positioned at the N-terminal or C-terminal end of the at least one costimulatory polypeptide comprising at least one 4-1BBL ectodomain, as this design allows a proper inherent trimerization of the 4-1BBL ligand. In other embodiments, the trimerization domain may be positioned between individual s4-1BBL domains in triple polypeptides.

Notably, no non-human sequences were incorporated into the new polypeptides and consequently adverse side effects are expected to be minor, which is of utmost importance for clinical and therapeutic applications. If artificial elements, for example, linkers or tags for purification, are used, these can be easily positioned in a polypeptide such that the artificial elements can be cleaved away after expression and purification. When expressed in an OV, the fully human core sequence of 4-1BBL can be elegantly expressed and presented to an immune cell without the need of a non-human sequence, if desired.

According to certain embodiments of the various aspects of the present invention, the polypeptides as disclosed herein may comprise a leader sequence. Leader sequences in terms of the present invention include all sequences, which are known to be involved in cellular trafficking and involve also all sequences that are known as "signal peptides" or "signal sequences" from the state of the art. An exemplary leader sequence is represented in SEQ ID NO: 17. Recombinant protein production may always be challenged with ensuring the correct folding and processing of the protein, such as post-translational modifications, to be produced. The folding and processing of proteins may be dependent on the cell compartment where the desired protein ends up. To ensure the correct guide of the desired protein, leader sequences may be used.

A leader sequence may thus fulfill the purpose of guaranteeing correct intracellular trafficking inside a host cell and/or correct targeting of a protein to the secretory pathway. This has the advantage that a protein or polypeptide comprising such a leader sequence will be correctly processed and/or post-translationally modified and/or will be correctly shuffled outside the cell in its soluble and correctly folded and thus functional form. Leader sequences will usually be adapted to a host cell of interest chosen for expression and/or production of a polypeptide or an OV of the present disclosure. The expression in eukaryotic host systems may have the advantage that the system mimics the natural folding of proteins derived from eukaryotic sequences. The use of a suitable leader sequence can also ensure correct folding in bacterial expression system, e.g. the leader sequence known as OmpA guides a protein of interest to the periplasm in prokaryotes where disulfide bridges are formed and where chaperones are present to assist in a proper protein folding necessary for correct protein/enzymatic function. Another critical point in recombinant protein production may be the glycosylation of eukaryotic proteins. A suitable leader sequence can also ensure the correct trafficking of the proteins to cell compartments where post-translational modifications, such as glycosylation, can take place.

Bacterial and/or eukaryotic expression systems for the various polypeptides of the present invention leader sequences may also be attached to a sequence encoding a polypeptide of interest, to guarantee that a protein is secreted outside the cell or to the periplasm of a bacterial cell to obtain soluble and correctly folded, and thus functional, polypeptides according to the present invention. Suitable leader and/or secretory signals or sequences are known to the skilled person for established expression systems. One further example (cf. **Figures 1A** and **2A****)** is the human IgKappa-light chain leader sequence, but, in essence, any leader sequence of a secreted protein having been demonstrated to be functional in secretion of a recombinant protein in a cellular system of interest may be used.

In certain embodiments of the various aspects of the present invention,, the costimulatory polypeptide may comprise at least one linker, in particular embodiments wherein the at least one linker comprises or consists of a sequence individually selected from the group consisting of SEQ ID NOs: 9 to 16, or a sequence having at least 90%, 91%, 92%, 93%, 94%, 95%, 96%, 97%, 98% or 99% sequence identity to any one of the sequences SEQ ID NOs: 9 to 16, or a nucleic acid sequence encoding the same, and/or wherein the costimulatory polypeptide , or the sequence encoding the same. In certain embodiments of the various aspects of the present invention, the costimulatory polypeptide additionally comprises at least one affinity tag and/or at least one protease cleavage tag and/or at least one inhibitory domain and/or at least one leader sequence. Linker sequences are well known to the skilled person and represent connecting elements in fusion proteins and multidomain polypeptides. Empirically, they are generally classified into three categories according to their structures: flexible linkers, rigid linkers, and *in vivo* cleavable linkers. All of these linkers may be used at the various positions, alone or in combination, in the various polypeptides as disclosed herein. Based on the inherent nature of a linker, the linker should be immunologically inert and/or removable (e.g. by defined proteases and/or chemicals). It is rather the function of a linker to improve folding and stability of the actual fusion protein or multidomain effector and/or to guarantee a correct spacing of the individual domains and thus a proper function thereof. The most commonly used flexible linkers have sequences consisting primarily of stretches of Gly and Ser residues ("GS" linker). Exemplary linkers of this kind are provided for with SEQ ID NOs: 9 to 16. Usually, these individual linker elements can be used in series (for example, 3x the sequence of SEQ IN NO: 11 in a row. A list of linkers as known to the skilled person and as suitable according to the present disclosure can be found in Chen et al. *supra.* The skilled person can adapt the length of the linker sequence based on the intended spacing properties as of interest to separate two functional moieties in a multi-domain polypeptide.

According to the various embodiments disclosed herein, a polypeptide encoding a costimulatory polypeptide according to the present invention may thus comprise at least one sequence encoding a linker providing a spacer function to properly separate the individual domains of a polypeptide, including at least one 4-1BBL encoding sequence and optionally at least one tag, at least one leader sequence, at least one inhibitory domain, at least one trimerization domain etc.

In certain embodiments according to the various aspects disclosed herein, the at least one costimulatory polypeptide comprising at least one 4-1BBL ectodomain may additionally comprise another effector domain provided as a fusion protein, optionally a cleavable fusion protein, together with the at least one costimulatory polypeptide comprising at least one 4-1BBL ectodomain of the present invention. For example, an effector domain may be selected from the group consisting of cancer targeting domains (capable of binding to a tumor-specific structure and/or-overexpressed surface ligand), an additional co-stimulatory signal to T- and/or NK-cells, a domain mediating prevention of inhibitory signaling (e.g. PD-1/PD-L1 interference), prodrug converters, toxins, reporter moieties for diagnostic/imaging purposes, therapeutic proteins, cytokines and chemokines, or a Fc-domain enhancing half-life time of a polypeptide, or combinations thereof.

As noted above certain embodiments of the aspects as disclosed herein may also comprise the use of at least one affinity tag. Affinity tags are generally fused to a recombinantly produced protein and can be used for protein purification to remove the protein of interest specifically from the crude cell culture broth. Examples for affinity tags well known in the art are chitin binding protein (CBP), maltose binding protein (MBP), Strep-tag and glutathione-S-transferase (GST). The poly(His) tag is commonly used and binds to metal matrices. Besides affinity tags, a variety of different protein tags are known which serve several functions such as marking the protein of interest for subsequent analysis, offering a solubilization tag for bacterial cell culture or marking the protein of interest via a fluorescence tag. Affinity tags as well as all other known protein tags can be easily removed from the product by enzymatic cleavage with proteases, in case a protease cleavage site is included into a polypeptide of interest. This may be preferable for therapeutic applications of the polypeptides as disclosed herein in view of the fact that the at least one tag first guarantees the purification of a s4-1BBL polypeptide to high purity, but the tag, as such usually not being of human origin, can then be removed from the recombinant polypeptide before a therapeutic use.

Proteases can be commonly used to reprocess the protein of interest and to remove tags, linkers, leader sequences or other specific polypeptide sequences from the protein of interest. For this purpose, protease cleavage sites may be incorporated in certain embodiments of the aspects as disclosed herein. A suitable protease may be selected from the group consisting of Arg-C proteinase, Asp-N endopeptidase, BNPS-Skatole, CNBr. Chemotrypsin, Clostripain, Glutamyl endopeptidase, LysC, LysN, Pepsin, Proline-edopeptidase, Proteinase K, Staphylococcal peptidase, Thermolysin, Trypsin, Caspase1, Caspase10, Caspase2, Caspase3, Caspase4, Caspase5, Caspase6, Caspase7, Caspase8, Caspase9, Enterokinase, Factor Xa, GranzymeB, Thrombin, Tobacco etch virus protease, SUMO-specific protease Ulp1p (scUIp1), ubiquitin (Ub), NEDD8, scAtg4, Atg8, xIUSP2, bdSENP1 and bdNEDP1 from *Brachypodium distachyon* (bd), as well as ssNEDP1 from salmon (*Salmo salar*)*.* The corresponding protein cleavage sites can be predicted with various available bioinformatics tools well known in the art.

In embodiments where the costimulatory polypeptide of the present invention is expresses as part of an OV, other protease recognition sites can be inserted, such as a capsid cleavage site as occurring in many naturally occurring viral polyproteins, which are needed for a processing of a virus by host cell and viral proteases. This effect can be mimicked by using a capsid cleavage site as artificial tag in any of the polypeptides as used herein, and, preferably, in an OV, as this will allow the processing inside a host cell of interest.

In yet another embodiment of the disclosed aspects, the costimulatory polypeptide may be encoded by a nucleic acid sequence selected from the group consisting of SEQ ID NOs: 47, 49, 51, 53, 55, 57, 59, 61, 63, 65, 67, 69, 71, 73, 75, 77 or 79 or a sequence having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to any one of the sequences SEQ ID NOs: 47, 49, 51, 53, 55, 57, 59, 61, 63, 65, 67, 69, 71, 73, 75, 77 or 79.

In certain embodiments of the various aspects of the present invention, the costimulatory polypeptide may comprise a Kozak sequence preceding the coding sequence to enhance protein translation by providing a suitable protein translation initiation site for the mRNA as encoded. An exemplary Kozak sequence is shown in SEQ ID NO: 80.

Notably, all nucleic acid sequences encoding at least one costimulatory 4-1BBL ectodomain polypeptide , either as part of a nucleic acid vector, or as part of an OV, may be codon optimized or at least partially codon optimized, preferably for the codon usage of a human cell in the case of an OV. For other recombinant expression systems for producing at least one costimulatory 4-1 BBL ectodomain polypeptide , it may also be preferable to choose the codon usage of the host cell of interest to obtain an optimum transcription and translation to avoid the commonly known phenomenon that rare codons in a given host cell may significantly hamper translation.

In a second aspect of the present invention, there may be provided a vector comprising a nucleic acid sequence encoding a costimulatory polypeptide according to the various aspects of the present invention.

A vector according to the present invention may be selected from any suitable vector for usage with the chosen expression system, such as bacterial, mammalian, plant, insect cell or yeast cell systems. Suitable vector systems for a variety of expression systems are well known in the art and a variety of suitable vectors as tested are disclosed in the attached sequence listing.

At least one vector comprising a nucleic acid sequence encoding a costimulatory polypeptide may be used to transfer the polypeptides according to the present invention into a host cell for recombinant protein production or for delivering the co-stimulatory polypeptides to a cell of interest. If a cell of interest should be targeted, this cell may be a tumor cell and the delivering vector may be an oncolytic virus.

In one embodiment of the above aspect, the vector may comprise a sequence encoding an expression vector, or an oncolytic virus, wherein the oncolytic virus may be selected from the group consisting of a vaccinia virus, a myxoma virus, an adenovirus, a herpes virus, a reovirus, a Zika virus, a vesicular stomatitis virus, a parvovirus, a poliovirus, an influenza virus, an arenavirus, a coxsackie virus, a semliki forest virus, a sindbis virus, a maraba virus, a seneca valley virus, a Newcastle disease virus, a mumps virus, and a measles virus, or combinations and/or chimera thereof.

As introduced above, cancer immuno-therapy is presently a promising new therapeutic strategy. Generally, it relies on the activation and arming of the immune system against tumors/cancers, as it is a feature of almost all cancers to escape from the immune system. There are many different approaches to address this problem, wherein oncolytic virus therapy (OVT) is one of the most encouraging technologies for providing new therapeutics. Oncolytic viruses (OVs) offer several advantages in cancer therapy as they have the dual function to activate the immune system to recognize and attack cancerous cells. Further, OVs may directly infiltrate and specifically kill cancer and cancerous cells.

According to the present invention, the OVs can be equipped with the costimulatory polypeptides of the present invention as functional effector or "payload". To this end, T cells can be specifically activated and targeted to malignant tissue.

An OV according to the various aspects and embodiments disclosed herein will usually be an attenuated virus in comparison to the cognate wild-type virus, i.e., an attenuated virus lacking virulence and pathogenicity traits, but retaining its capacity to replicate and specifically target cells in an *in vivo* or *in vitro* context.

In another embodiment of the above second aspect, there is thus provided an oncolytic virus encoded by a vector according to the second aspect, wherein the oncolytic virus may comprise a costimulatory polypeptide as payload according to the first aspect and embodiments thereof of the present invention. The OV will thus present the at least one 4-1BBL costimulatory polypeptide to the immune system to synergistically activate the immune system together with the inherent cancer targeting capabilities of the OV. Several different OVs harboring and expressing at least one 4-1BBL costimulatory polypeptide are detailed herein below and in the sequence listing provided. In case the OV is originating from a measles virus (MV), this OV vehicle naturally targets the host cell receptors CD46, SLAM/CD150 and PVRL4 (nectin-4). Therefore, MV (without any payload) is already latently oncotropic in nature and possesses oncolytic properties by syncytia formation and lytic effects. In artificial MVs as disclosed herein, in particular when combined with at least one 4-1BBL costimulatory polypeptide of the present invention, allows the provision of a highly specific agent for viral-based oncolytic therapy. MV is generally of round shape but shows pleomorphism, i.e. the appearance of two or more distinctly different forms in the life cycle of some organisms. It contains single-stranded RNA of negative polarity. It is an enveloped virus with non-segmented genome. Replicating recombinant MV has been shown to represent a valuable backbone or vector for providing safe and efficient viral vectored vaccines against several emerging infectious diseases (Reisinger et al., Lancet, 2019, 392(10165):2718-2727; Schrauf et al., Front. Immunol., 2020, 11:592). Using MV as cargo or vector for oncolytic therapy thus may build upon experience gained for MV vaccines and MV-based viral vectored vaccines as established over many decades. Additionally, further properties, particularly a tumor tropism and thus tumor-specificity, can be additionally exploited during OV based on a MV structure or vector.

Generally, other types of OVs can be used as vehicles or backbones for transporting additional payloads to a tumor target site. The primary mechanism of tumor/cancer targeting will depend on the properties of the OV usually representing a modified and attenuated version of a naturally occurring virus. This OV represents the vector or backbone for introducing at least one payload. In certain embodiments, the OV of interest may comprise more than one payload, wherein at least one of the payloads is at least one 4-1BBL costimulatory polypeptide of the present invention. For example, a vaccinia virus (VV) may be used as OV. As for MV, the virus biology and life cycle of VV is well understood and preceding data for using VV in vaccination studies exist, which may expedite the development of VV-based OVs equipped with at least one 4-1BBL polypeptide according to the present invention (cf. Kim et al., Host lymphodepletion enhances the therapeutic activity of an oncolytic vaccinia virus expressing 4-1BB ligand. Cancer Res. 2009;69:8516-25). Thymidine kinase deletion can prevent VV (a DNA virus) replication in cells with a low nucleotide pool. DNA viruses usually rely upon many different mechanisms to increase dNTP levels in infected cells, because the low concentration of dNTPs found in non-cycling cells can inhibit virus replication. By disrupting the virus-encoded gene(s) that normally promote dNTP biosynthesis, oncolytic versions of, for example, VV can be created that replicate selectively in cancer cells (Irwin et al., Front. Oncol., 2017, 7:229).

The choice of the OV backbone when used in the context of the present invention will usually depend on the indication, i.e., the type of tumor/cancer to be targeted in view of the fact that each OV originates from a naturally occurring virus with a specific patho-biology and, in turn, tumor specificity. For example, adenovirus may be configured to selectively target cells with aberrant mRNA transport due to adenovirus E1B protein deletion to be used to combat head and neck tumors, coxsackie virus may be used to target DAF (intracellular adhesion molecule 1), which is overexpressed in cancer cells, so that this OV can be used to target melanomas, herpes virus can be modified by a ICP34.5 deletion to restrict replication to cells with a (highly) active replication, as it is the case for many cancer cells, including melanoma cells, glioma, astrocytoma, glioblastoma, squamous cell carcinoma of head and neck etc. Newcastle disease virus (NDV) as OV also can built upon around fifty years of clinical application giving witness to the high safety profile of this biological agent. Since NDV as avian virus has neither adverse effects on human cells nor any pathology, it can be used as OV in cancer patients. NDV is naturally unable to replicate in interferon-responsive cells and can be equipped in line with the present disclosure to target, for example, metastatic cancers. Reoviruses (mammalian orthoreoviruses) are non-enveloped viruses that contain segmented dsRNA. Clinical manifestations associated with natural reovirus infection make this virus an ideal candidate for development for cancer virotherapy that can be used even in immunocompetent and immunocompromised patients. Oncolysis relies on the intrinsic capacity of reovirus to kill cancer cells. Reoviruses modulate interferon (IFN) responses during an infection with the virus, wherein the IFN-1 response is a key element of the innate immune response to reovirus. Within infected cells, viral RNAs are detected by cellular pattern recognition receptors (PRRs), including retinoic acid inducible gene-I (RIG-I), melanoma differentiation-associated protein 5 (MDA5), toll-like receptors, and the dsRNA-activated protein kinase R (PKR) ultimately leading to an NF-κB) and activator protein-1 (AP-1) activation to induce expression and secretion of IFN-1 (IFN-α and IFN-β). Additionally, the EGFR signaling pathway via Ras plays a critical role in growth and survival of reoviruses during natural infections (Philipps et al., Oncolytic Virother., 2018, 7: 53-63). In OV clinical trials, reovirus is thus used to target metastatic cancers and glioma. Seneca valley virus also has an inherent capacity to target tumor cells. This virus of the *Picornaviridae* family has a high tropism towards neuroendocrine expressing tumor cells has been shown to induce cytotoxicity in tumors expressing neuroendocrine features, such as synaptophysin, chromogranin A, and neuron-specific enolase, in several in vitro and in vivo models (Burke, Oncolytic Virother., 2016, 5: 81-89).

Therefore, various OVs to be used according the present invention are known to the skilled person. These OVs can be individually equipped with the highly active 4-1BBL costimulatory polypeptides according to the present invention to favourably stimulate 4-1BB/CD137 signaling to achieve a co-stimulation of T-cells to break cancer immune evasion of cancer cells and tumors by additionally targeting a T-cell response to a cancer/tumor to be treated.

In certain embodiments of the present invention, the vectors and polypeptides disclosed herein may be used in combination with adoptive T cell transfer, such as infusion of isolated and *ex vivo* cultivated tumor-infiltrating lymphocytes (TIL) and/or a CAR T-cell strategy. As for the combination with an OV, this strategy allows the combination of the adoptively transferred T-cells (that can be genetically engineered, e.g. to express a chimeric antigen receptor (CAR) and thus specifically directed towards antigens on cancer/tumor cells) with the costimulatory effects of the 4-1BBL polypeptides of the present invention to additionally activate the endogenous immune response of a patient in a specific manner. To this end, blood cancers like lymphoma and leukemia can be treated, as well as solid tumors. To this end, an activation with the specific 4-1BBL costimulatory polypeptides may assist in breaking the immuno-evasive tumor microenvironment so that CAR-T-cells, which usually do not traffic efficiently into solid tumors, in particular, cold tumors, can reach antigens expressed on target cancer cells of a solid tumor/cancer.

Moreover, the 4-1BBL costimulatory polypeptides of the present invention can be used during the *ex vivo* cultivation of T cells and/or NK cells in preparation of an adoptive cell transfer to improve their therapeutic potential.

In a third aspect of the present invention, there is provided a method of producing a costimulatory polypeptide, wherein the method comprises: (i) providing a vector according to the above second aspect and embodiments thereof encoding a costimulatory polypeptide, (ii) introducing the vector into a host cell; (iii) culturing the host cell under conditions suitable for expression of the costimulatory polypeptide; (iv) optionally: isolating and purifying the costimulatory polypeptide ; and (v) obtaining the costimulatory 4-1BBL polypeptide .

In a fourth aspect of the present invention, there is provided a method of producing an oncolytic virus expressing a costimulatory polypeptide, wherein the method comprises: (i) providing a vector as defined according to the second aspect and embodiments thereof encoding an oncolytic virus comprising the costimulatory polypeptide, (ii) introducing the vector into a host cell, (iii) culturing the host cell under conditions suitable for expression and thus replication of the oncolytic virus, (iv) optionally: rescuing the oncolytic virus; (v) optionally: purifying the oncolytic virus as obtained in step (iii) or (iv); and (vi) obtaining an oncolytic virus.

Techniques for general cloning of the above polypeptides are generally available to the skilled person (Green and Sambrook, Molecular Cloning, A Laboratory Manuel 4th edition, last updated 18 November 2014).

Rescue techniques are needed in the context of certain OVs in view of the fact that the artificial OVs comprising a payload have to be obtained in an active, i.e. infectious and replicative form. These rescue techniques depend on the biology of the virus an OV originates from. For certain OVs as disclosed herein, rescue techniques are available to the skilled person for the various DNA- and RNA-viruses as disclosed herein (cf. WO 2017/109222, or, for CMV RNA polymerase II promoter containing plasmids, WO 2012/022495). Further rescue systems are available for various viruses to the skilled person to obtain functional recombinant infectious virus particles including a payload as disclosed herein. Generally, the rescue technique will depend on the type of genome and the biology of a virus of interest to be used as OV.

Further, a costimulatory polypeptide and/or an oncolytic virus comprising such a polypeptide according to the present invention can be further purified to obtain a high degree of purity needed for therapeutic applications. For costimulatory 4-1BBL polypeptides as recombinant protein, various purification strategies, *inter alia,* relying on the use of affinity tags etc., are available for the skilled person.

Purification strategies for oncolytic viruses are also available to the skilled person in the relevant technical field. To this end, it is pointed out that the purification will largely depend on the diameter and/or the physico-chemical properties (in particular, of the surface exposed parts) of an OV of interest. In particular, for MV as a large and pleomorphic virus, purification has been difficult. Meanwhile, suitable purification strategies are also available for this sterically demanding virus (cf. WO 2019/238919).

Based on the functional data generated with the various costimulatory polypeptides according to the present invention based on the core and soluble TNF homology domain, either alone, or as part of an OV, it could be shown that these polypeptides indeed have superior functionality in T-cell activation (see Examples below) and thus can be used for a broad range of cancer therapeutic strategies in view of the central role of the 4-1BBL signaling in T-cell activation urgently needed as further line of anti-tumor activity.

In a fifth aspect of the present invention, there is provided a pharmaceutical composition which may comprise a costimulatory polypeptide according to the first aspect and embodiments thereof as defined above and/or an oncolytic virus according to the second aspect and embodiments thereof as defined above, and/or as obtained according to the fourth aspect of the present invention as defined above.

Either an isolated costimulatory polypeptide according to the various aspects of the present invention, or an OV expressing and presenting the same can be used as a therapeutic agent, preferably in combination with suitable pharmaceutically acceptable carriers and/or excipients and within a pharmaceutical formulation guaranteeing stability and safety of the relevant biopharmaceutical. Generally, the pharmaceutical composition can be provided as solid or fluid composition, for example, as a lyophilized product to be dissolved in an aqueous solvent, or as liquid formulation already comprising stabilizing and pharmaceutically acceptable buffers, excipients etc.

As could be demonstrated, the various polypeptides and OVs according to the present invention have a 4-1BB receptor signaling modulating activity and, in combination with an OV, a specific cancer-tropism as mediated by the OV.

In a sixth aspect of the present invention, there is thus provided a costimulatory polypeptide according to the first aspect and embodiments thereof as defined above, and/or an oncolytic virus according to the second aspect and embodiments thereof as defined above, and/or as obtained according to the fourth aspect as defined above which can be used in a method of treating cancer.

There is thus provided a method of treating a cancer in a subject by providing an effective amount of a costimulatory polypeptide and/or an oncolytic virus expressing a costimulatory polypeptide of the present invention. When combined with the OV technology, a specific targeting and expression of the at least one 4-1BBL can be obtained, which may be of particular relevance to break the dormant state of, for example, cold tumors that are difficult to target in therapy.

Further provided is the use of an effective amount of a costimulatory polypeptide and/or an oncolytic virus expressing a costimulatory polypeptide of the present invention in a method of treating cancer in a subject in need thereof.

Further provided is use of an effective amount of a costimulatory polypeptide and/or an oncolytic virus expressing a costimulatory polypeptide of the present invention in the manufacture of a medicament for the treatment of cancer in a subject in need thereof.

In one embodiment of the foregoing: (i) methods of treating cancer, (ii) uses in a method of treating cancer, or (iii) uses in the manufacture of a medicament for treating cancer, the cancer may be selected from bladder cancer, breast cancer, prostate cancer, basal cell carcinoma, biliary tract cancer, bone cancer, brain and central nervous system cancer (e.g., glioma), adenocarcinomas, lung cancer, cervical cancer, choriocarcinoma, colon and rectum cancer, connective tissue cancer, cancer of the digestive system; cancer of the small intestine and cecum; endometrial cancer, esophageal cancer; eye cancer; cancer of the head and neck; gastric cancer; intra-epithelial neoplasm; kidney cancer; larynx cancer; leukemia; liver cancer; gall bladder cancer lung cancer (e.g., small cell and non-small cell); lymphoma including Hodgkin's and Non-Hodgkin's lymphoma; melanoma; myeloma, neuroblastoma, oral cavity cancer (e.g., lip, tongue, mouth, and pharynx); cancer of the salivary glands; ovarian cancer; pancreatic cancer, retinoblastoma; rhabdomyosarcoma; rectal cancer, renal cancer, cancer of the respiratory system; sarcoma, skin cancer; stomach cancer, testicular cancer, thyroid cancer; uterine cancer, cancer of the urinary system, as well as other carcinomas and sarcomas.

In another embodiment of the foregoing: (i) methods of treating cancer, (ii) uses in a method of treating cancer, or (iii) uses in the manufacture of a medicament for treating cancer, the cancer is a cold tumor or a hot tumor. As detailed above, 4-1BBL-mediated signaling can specifically convert a dormant cold tumor so that the cancer cells can be additionally attacked by the T-cells of a patient so that immuno-evasion can be broken.

Various drug delivery techniques and vehicles are known to the skilled person to deliver the at least one costimulatory polypeptide to a cancerous site in a subject in need thereof, including, for example, direct injections, but also compounds based on synthetic polymers, proteins, lipids, and organic and inorganic particles approved for cancer therapeutics, which compounds or vehicles may be in the nanoscale. Compared with the direct administration of bare chemo-drugs, drug encapsulation in a vehicle can offer a number of advantages, such as protection from degradation in the bloodstream, better drug solubility, enhanced drug stability, targeted drug delivery, decreased toxic side effects and improved pharmacokinetic and pharmacodynamic drug properties.

To prepare pharmaceutical or sterile compositions of the polypeptides and oncolytic viruses, the polypeptide or oncolytic virus is admixed with a pharmaceutically acceptable carrier or excipient. See, e.g., Remington's Pharmaceutical Sciences and U.S. Pharmacopeia: National Formulary, Mack Publishing Company, Easton, PA (1984).

Formulations of therapeutic and diagnostic agents may be prepared by mixing with acceptable carriers, excipients, or stabilizers in the form of, e.g., lyophilized powders, slurries, aqueous solutions or suspensions (see, e.g., Hardman, et al. (2001) Goodman and Gilman's The Pharmacological Basis of Therapeutics, McGraw-Hill, New York, NY; Gennaro (2000) Remington: The Science and Practice of Pharmacy, Lippincott, Williams, and Wilkins, New York, NY; Avis, et al. (eds.) (1993) Pharmaceutical Dosage Forms: Parenteral Medications, Marcel Dekker, NY; Lieberman, et al. (eds.) (1990) Pharmaceutical Dosage Forms: Tablets, Marcel Dekker, NY; Lieberman, et al. (eds.) (1990) Pharmaceutical Dosage Forms: Disperse Systems, Marcel Dekker, NY; Weiner and Kotkoskie (2000) Excipient Toxicity and Safety, Marcel Dekker, Inc., New York, NY).

Toxicity and therapeutic efficacy of the antibody compositions, administered alone or in combination with another agent, can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index (LD50/ ED50). In particular aspects, polypeptides and oncolytic viruses exhibiting high therapeutic indices are desirable. The data obtained from these cell culture assays and animal studies can be used in formulating a range of dosage for use in human. The dosage of such compounds lies preferably within a range of circulating concentrations that include the ED50 with little or no toxicity. The dosage may vary within this range depending upon the dosage form employed and the route of administration.

In a further embodiment, a composition comprising a polypeptide or oncolytic virus disclosed herein is administered to a subject in accordance with the Physicians' Desk Reference 2003 (Thomson Healthcare; 57th edition (November 1, 2002)).

The mode of administration can vary. Suitable routes of administration include parenteral; intramuscular, subcutaneous, intradermal, intramedullary, intrathecal, direct intraventricular, intravenous, intraperitoneal, or intra-arterial.

In particular embodiments, the anti-target antibody or antigen binding fragment thereof can be administered by an invasive route such as by injection (see above). In further embodiments of the invention, a polypeptide, oncolytic virus, or pharmaceutical composition thereof, is administered intravenously, subcutaneously, intramuscularly, intra-arterially, or intratumorally.

Compositions can be administered with medical devices known in the art. For example, a pharmaceutical composition of the invention can be administered by injection with a hypodermic needle, including, e.g., a prefilled syringe or autoinjector.

The pharmaceutical compositions disclosed herein may also be administered by infusion.

Alternately, one may administer the polypeptide or oncolytic virus in a local rather than systemic manner, for example, via injection directly into a tumor, often in a depot or sustained release formulation.

The administration regimen depends on several factors, including the serum or tissue turnover rate of the polypeptide or oncolytic virus, the level of symptoms, the immunogenicity of the polypeptide or oncolytic virus, and the accessibility of the target cells in the biological matrix. Preferably, the administration regimen delivers sufficient polypeptide or oncolytic virus to effect improvement in the target disease state, while simultaneously minimizing undesired side effects. Accordingly, the amount of biologic delivered depends in part on the particular polypeptide or oncolytic virus and the severity of the condition being treated.

Determination of the appropriate dose is made by the clinician, e.g., using parameters or factors known or suspected in the art to affect treatment. Generally, the dose begins with an amount somewhat less than the optimum dose and it is increased by small increments thereafter until the desired or optimum effect is achieved relative to any negative side effects. Important diagnostic measures include those of symptoms of, e.g., the inflammation or level of inflammatory cytokines produced.

A convenient and fully site-specific technique to present at least one costimulatory polypeptide to the immune system of a subject in need thereof is the delivery of the costimulatory polypeptide as part of an OV as payload. This combination not only allows 4-1BB receptor interaction and signaling modulation, it further allows targeting a polypeptide comprising, or a nucleic acid encoding, at least one costimulatory polypeptide of this invention immediately to a cancer in a highly site-specific manner in view of the tumor tropism of the OV of interest.

In an additional aspect of the present invention, there is provided a kit, wherein the kit may comprise a costimulatory polypeptide according to the above first aspect or an embodiment thereof, or an oncolytic virus expressing a costimulatory polypeptide according to above second aspect or an embodiment thereof, optionally wherein the kit comprising further reagents.

A kit may thus comprise suitable buffers, reagents and the like for solubilizing and/or stabilizing and/or activating the costimulatory polypeptide according to the methods and uses as disclosed herein. The kit may further comprise at least one protease and reagents for cleaving a tag or sequence within the polypeptides of the present invention in case these polypeptides comprise a protease cleavage site.

Finally, there is provided the use of an effective amount of costimulatory polypeptide according to the above first aspect or an embodiment thereof, or of an oncolytic virus expressing a costimulatory polypeptide according to the above second aspect or an embodiment thereof in an *in vitro* method for stimulating 4-1BB expressing cells.

The present invention will now be further illustrated with the help of the following nonlimiting Examples.

### Examples:

### Example 1: Polypeptide design and expression of polypeptides

Six different costimulatory polypeptides according to the invention were designed comprising either one or three 4-1BBL domains according to SEQ ID NO: 1 or 4. For comparing these new polypeptides in functional assays with available 4-1BBL agents, four polypeptides comprising an scFv from one of two fully human anti-4-1BB/CD137 agonist mAbs (MAb1 and MAb2) for comparison, and optionally a trimerization domain and a polypeptide comprising one 4-1BBL domain and a trimerization domain of chicken tenascin were analyzed and compared to the costimulatory polypeptides according to the invention. The domains of the polypeptides are listed in **Table 1,** a graphical representation can be found in the listed figures. It has to be noted that each position and each domain, tag, leader-sequence, linker-sequence or promotor sequence can be rearranged or interchanged for constructing a highly active costimulatory polypeptide.

**Table 1: Domains present in costimulatory polypeptides according to the invention and design of comparison polypeptides**

| **Polypeptide Name** | **SEQ ID NO** | **Linker** | **Tags** | **Effector domain** | **Trimerization domain** | **Graphical representation** |
|---|---|---|---|---|---|---|
| MAb1-scFv (comparison 1) | | SEQ ID NO: 9 | His-Tag, Strep-Tag | MAb1 scFv | - | Fig 1A |
| MAb1-scFv-Tri_{XVIII} (comparison 1) | | SEQ ID NO: 9 | His-Tag, Strep-Tag | MAb1 scFv | SEQ ID NO: 7 | Fig. 1A |
| MAb2-scFv | | SEQ ID NO: 9 | His-Tag, Strep-Tag | MAb2 scFv | - | Fig. 2A |
| MAb2-scFv-Tri_{XVIII} (comparison 2) | | SEQ ID NO: 9 | His-Tag, Strep-Tag | MAb2 scFv | SEQ ID NO: 7 | Fig. 2A |
| s4-1BBL | 48 | SEQ ID NO: 9, 12 | His-Tag, Strep-Tag | 4-1BBL (SEQ ID NO: 4) | - | Fig. 3A |
| s4-1 BBL-Tri_{XVIII} | 54 | SEQ ID NO: 9, 12 | His-Tag, Strep-Tag | 4-1BBL (SEQ ID NO: 4) | SEQ ID NO: 7 | Fig. 4A |
| s4-1 BBL-Tri_{XVIII}^{LL} | 72 | SEQ ID NO:9, 12, 15 | His-Tag, Strep-Tag | 4-1BBL (SEQ ID NO: 4) | SEQ ID NO: 7 | Fig. 3A, Fig. 4A, Fig 5A, |
| s4-1 BBL-Trixv | 52 | SEQ ID NO: 9, 12 | His-Tag, Strep-Tag | 4-1BBL (SEQ ID NO: 4) | SEQ ID NO: 8 | Fig.: 4A |
| Triple-s4-1-BBL | 61 | SEQ ID NO: 9, 11, 12 | His-Tag, Strep-Tag | 4-1 BBL (x3) (SEQ ID NO: 4) | - | Fig.: 5A |
| Triple-s4-1 BBL-Tri_{XVIII} | 66 | SEQ ID NO: 9, 11, 12 | His-Tag, Strep-Tag | 4-1 BBL (x3) (SEQ ID NO: 4) | SEQ ID NO: 7 | Fig.: 5A |
| s4-1 BBL-TNC (comparison 3) | 86 | SEQ ID NO: | | 4-1BBL (SEQ ID NO: 4) | SEQ ID NO: 7 | Fig. 6A |
| s4-1 BBL-short (sh) minimal polypeptide | 76 | SEQ ID NO: 9 | His-Tag, Strep-Tag | 4-1 BBL minimum or core sequence (SEQ ID NO: 1) | SEQ ID NO: 7 | |

### 1. Polypeptide design and gene synthesis of monomeric MAb1-based anti-4-1BB scFv

### a) MAb1

The protein sequence of MAb1, which binds 4-1BB, was taken from the KEGG database. Afterwards, *in silico* design of a corresponding single-chain antibody (scFv) was conducted: To enable secretion of the scFv, a signal peptide derived from the human Igκ light chain (SEQ ID NO: 91) was added to the N-terminus of the protein sequence, followed by the variable domain of the heavy chain (VH) sequence of MAb1, a (G₄S)₃-linker sequence (SEQ ID NO: 11), and the VL domain sequence of MAb1 for the MAb1-derived scFv. The protein sequence was back-translated into its corresponding human codon-optimized DNA sequence. A synthesized sequence encoding MAb1-scFv was used to get two single chain fragment polypeptides, namely MAb1-scFv and MAb1-scFv-Tri_{XVIII}, which additionally contains a trimerization domain. MAb1-scFvwas amplified via PCR. Thereby a sequence encoding a Strep-Tag and a HIS-Tag separated by a linker (SEQ ID NO: 9) were added at the C-terminus. Additionally, a BamHI restriction site was added separating the scFv from the Tags. Therefore, a first PCR was performed using the following primer pair: forward primer *MAb1-F-HindIII* (SEQ ID NO: 94) and the reverse primer *MAb1-B1* (SEQ ID NO: 95). Using the resulting product as a template a second PCR was performed with the same forward and *B2-NotI* (SEQ ID NO: 96) as reverse primer. Sequences encoding the Strep-Tag and the His-Tag were thereby included in the PCR reverse primers and added via PCR. The product was cloned into a pCEP4 expression vector using Hindlll and Notl restriction enzymes. At this step, it is possible to clone the resulting nucleic acid in any suitable expression vector for expression in a bacterial, plant and mammalian expression system. For the MAb1-scFv-Tri_{XVIII}, the trimerization domain had to be added via PCR. In a first PCR the MAb1 part was amplified using the forward primer *MAb1-F-HindIII* (SEQ ID NO: 94) and the reverse primer *MAb1-B* (SEQ ID NO: 97. The trimerization part was amplified from the MAb2-Tri-scFv nucleic acid sequence (see below) with the following primer pair: *MAb1-Tri-F* (SEQ ID NO: 98) as forward primer and *TriDom-B-BamHI* (SEQ ID NO: 99) as reverse primer. The obtained two PCR product were then joined in a third PCR using the forward primer of the MAb1 part and the reverse primer of the trimerization part. The product was cloned into the pCEP4 expression vector using Hindlll and BamHI restriction enzymes. Downstream of the BamHI site this vector already contains a Strep-Tag and a HIS-Tag sequence.

### b) MAb2

The protein sequence of MAb2 was taken from the KEGG database. Afterwards, *in silico* design of a corresponding single-chain antibody (scFv) was conducted. To enable secretion of the scFv, a signal peptide derived from the human Igκ light chain (SEQ ID NO: 91) was added to the N-terminus of the protein sequence, followed by the complementarity defining region (CDR) VH sequence of MAb2, a (G₄S)₃-linker sequence (SEQ ID NO: 11) and the CDR VL region sequence of MAb2 for the MAb2-derived scFv. The protein sequence was back-translated into its corresponding human codon-optimized DNA sequence. The PCR for cloning and amplification was conducted similarly to a) with the primers listed in SEQ ID NOs: 113 and 99, respectively.

### c) s4-1 BBL, s4-1 BBL-Tri_{XVIII}, s4-1BBL-Tri_{XVIII} and s4-1 BBL-Tri_{XV}

An expression plasmid encoding human 4-1BBL (Kober et al., Eur J Immunol. 2008 Oct; 38(10): 2678-2688) was used to generate two PCR-products encoding soluble (s) 4-1BBL proteins, namely s4-1BBL and s4-1BBL-Tri_{XVIII}^{LL}, which both contain a CD5 Leader sequence followed by a Strep-Tag and a HIS-Tag sequence, separated by a GGSGG linker upstream of the extracellular part of 4-1BBL (SEQ ID NO: 4). s4-1BBL-Tri_{XVIII}^{LL} additionally harbours the same trimerization domain that has been used for trimerization of MAb1-scFv and MAb2-scFv between the Tag sequences and the 4-1BBL extracellular region. The trimerization domain is separated from the tags by a long 25mer linker. In order to amplify 4-1BBL, the following primers were used: *Tags-4-1BBL-F1* (SEQ ID NO: 102) as forward primer and *4-1BB-L-B-NotI* (SEQ ID NO: 103) as reverse primer. Using the obtained product as a template, a second PCR was performed with the same reverse primer, but the following forward primer: *CD5L-Tags-F2-HindIII* (SEQ ID NO: 104). For the 4-1BBL-Tri_{XVIII}^{LL}, the trimerization domain had to be added via PCR. At first two PCR reactions had to be performed. The trimerization domain was amplified using the MAb2-Tri_{XVIII}-scFv as a template and the following primers: *Tags-TriDom-F1* (SEQ ID NO: 105) as forward and *TriDom-4-1BB-L-B* (SEQ ID NO: 106) as reverse primer. The 4-1BBL part was amplified using an already cloned 4-1BBL sequence as a template and the following primers: forward, TriDom-4-1BB-L-F (SEQ ID NO: 107) and *4-1BB-L-B-NotI* (SEQ ID NO: 103) as reverse primer. The obtained two PCR products were then joined in a third PCR using the *CD5L-Tags-F2-HindIII* (SEQ ID NO: 104) and the *4-1BB-L-B-NotI* (SEQ ID NO: 103) primers. For s4-1BBL-Tri_{XVIII}, the 25mer linker (SEQ ID NO: 15) was exchanged with a shorter linker sequence according to SEQ ID NO: 9. For s4-1BBL-Tri_{XV}, the trimerization domain was exchanged with a domain according to SEQ ID NO: 8. All four products (4-1BBL and 4-1BBL-Tri_{XVIII}^{LL}) were cloned into the pCEP4 expression vector using HindIII and Notl restriction enzymes.

### d) Triple-s4-1BBL and Triple-s4-1BBL-Tri_{XVIII}^{LL}

The polypeptide is based on the protein sequence of 4-1BBL (SEQ ID NO: 4) with the addition of different linker sequences (SEQ ID NOs: 9, 10 and 15) and a Strep- and His-Tag. The Triple-s4-1BBL-Tri_{XVIII} polypeptide additionally comprises a trimerization domain according to SEQ ID NO: 7. The full-length polypeptide has a sequence according to SEQ ID NO: 64 (Triple-s4-1BBL) or SEQ ID NO: 74 (Triple-s4-1BBL-Tri_{XVIII}^{LL}).

Synthesized sequences encoding human Triple-s4-1 BBL and Triple-s4-1 BBL-Tri_{XVIII}^{LL} were amplified via PCR using the following primer pair: Triple-s4-1BBL-F-HindIII (SEQ ID NO: 108) as forward primer and Triple-s4-1BBL-B-NotI (SEQ ID NO: 109) as reverse primer. Triple-s4-1BBL (SEQ ID NO: 64) and Triple-s4-1BBL-Tri_{XVIII}^{LL} (SEQ ID NO: 74) were cloned into the pCEP4 expression vector using HindIII and NotI restriction enzymes.

### e) s4-1BBL-TNC

Another comparison example is the polypeptide of s4-1BBL with a trimerization domain of chicken tenascin (TNC).

An expression plasmid encoding human s4-1BBL-Tri_{XVIII}^{LL} was used to generate the PCR-product encoding soluble (s) 4-1BBL-TNC protein, which contains a CD5 Leader sequence followed by a Strep-Tag and a HIS-Tag sequence, separated by a GGSGG linker, upstream of the extracellular part of 4-1BBL (SEQ ID NO:4). Between the Tag sequences and the 4-1BBL extracellular region lies the trimerization domain chicken tenascin-C (SEQ ID NO: 110). In order to generate s4-1BBL-TNC two PCR reactions had to be performed using s4-1BBL-Tri_{XVIII}^{LL} as a template and the following primers: in the first step *CD5L-Tags-F2-HindII* (SEQ ID NO: 104) is used as forward primer and *Tags-TNC-B* (SEQ ID NO: 111) is used as reverse primer. In the second PCR, *Tags-TNC-F* (SEQ ID NO: 112) is used as forward primer and 4-1BBL-B-NotI (SEQ ID NO: 103) is used as reverse primer. The obtained two PCR products were then joined in a third PCR using the *CD5L-Tags-F2-HindIII* and the *4-1BBL-B-NotI* primer pair (SEQ ID NOs: 104 and 103). s4-1BBL-TNC was cloned into the pCEP4 expression vector using Hindlll and Notl restriction enzymes, the nucleic acid sequence corresponds to SEQ ID NO: 83.

### 2. Expression of polypeptides

All expression plasmids listed above were transfected into HEK293T cells and were cultivated under conditions suitable for the expression of polypeptides. The expression system can be exchanged to any suitable expression system, such as bacterial, yeast, plant (including algae), insect cell-derived expression systems, or mammalian expression systems, including, for example, CHO, HEK293(T), HeLa, COS, Vero, NS0, U2OS, A549, HT1080, CAD, P19, NIH 3T3, L929, N2a, MCF-7, Y79, SO-Rb50, Hep G2, DUKX-X11, J558L, BHK, and Sp2/0 cells and their derivatives. For a survey, reference is made to Khan, Adv. Pharm. Bull., 2013, 3(2):257-263.

The cells were then harvested and the supernatants were taken for subsequent analysis. Similar protocols are available for intracellular expression in various systems. Polypeptides having a leader sequence (e.g., SEQ ID NO: 17) being compatible with a eukaryotic host cell of interest may be strongly preferred in case the relevant leader allows proper intracellular trafficking and secretion of the properly folded protein. Even though certain polypeptides as disclosed herein are perfectly suitable for expression in bacterial hosts, it may be favourable to express soluble and correctly folded proteins in eukaryotic cells to have full functionality. Generally, intracellular expression in bacteria and/or refolding of inclusion bodies may have certain drawbacks in case a eukaryotic, and particularly human, protein sequence to be expressed comprises glycosylation sites, which cannot be properly glycosylated in the bacterial host leading to less functional proteins upon bacterial expression. In view of the fact that the 4-1BBL polypeptides as designed do no longer comprise cysteine residues, inclusion body formation (when choosing a production in the bacterial cytoplasm, not the periplasm), another frequently encountered problem during cytoplasmic expression in bacteria and subsequent refolding to re-gain activity of a protein of interest can be avoided.

### Example 2: Binding assays

For testing the resulting supernatants, a Triple parameter reporter cell line (TPR) based on the human Jurkat JE6-1 T cell line was used (Jutz et al., 2016). This cell line harbors fluorescent reporter polypeptides to concomitantly assess the activity of three transcription factors, which play major roles during T cell activation: NF-κB::eCFP; NFAT::eGFP and AP-1::mCherry. The TPR reporter cells were transduced to stably express human 4-1BB. For the binding assays, 1x10⁵ 4-1BB expressing cells in a volume of 10 µl were incubated with 50 µl of the supernatants containing the products according to Table 1. As controls, cells that do not express 4-1BB, and a control-scFv were used. After an incubation time of 30 min at 4 °C, the samples are washed in FACS-Buffer (1xPBS, 0.5% FCS, 0.005% NaN₃) and stained with a biotinylated Strep-tag II mAb (GenScript, NJ) via adding 5 µl of a 1:150 dilution to each sample and incubation for 20 min on 4 °C. Afterwards, samples were washed again and incubated for another 20 min on 4 °C upon adding 5 µl of a 1:200 dilution of Streptavidin-PE (BD Pharmingen, San Diego, CA) to each sample. At last, samples were washed again and subsequently measured via flow cytometry, which was performed on a FACSCalibur flow cytometer (Becton Dickinson Immunocytometry System, San Jose, CA), using the CellQuest software. Data was analyzed with FlowJo (version 10.0.7, Tree Star, Ashland, OR) and Graphpad Prism (version 6, GraphPad Software, Inc., La Jolla, CA). The following results were obtained:

### 1. MAb1-scFV and MAb2 ScFv

The binding of MAb1-scFV is shown in **Figure 1C****.** By performing flow cytometry based binding studies, a specific binding of MAb1-scFv or MAb1-Tri_{XVIII}-scFv to 4-1BB-expressing cells could be confirmed, whereas these antibody fragments did not bind to control cells not expressing 4-1BB. The binding was dose-dependent and still detectable when using a 1:1600 dilution of both supernatants. A control single chain fragment (in this case Pembrolizumab-scFv), which was used as an additional control did not bind to 4-1BB-expressing cells and to the control cells.

The binding of MAb2-scFV is shown in **Figure 2B****.** By performing flow cytometry based binding studies, a specific binding of MAb2-scFv or MAb2-scFv-Tri_{XVIII} to 4-1BB-expressing cells was not detected. Furthermore, MAb2-scFv and MAb2-scFv-Tri_{XVIII} could not be detected in Western blot analysis, whereas the Ctrl-scFv (MAb1-scFv and MAb1-Tri_{XVIII}-scFv) could be seen as strong bands, which shows the Western blot to be working.

### 2. s4-1BBL and s4-1BBL-Tri_{XVIII}^{LL}

The binding of s4-1BBL and s4-1BBL-Tri_{XVIII}^{LL} can be seen in **Figure 3B****.** By performing flow cytometry based binding studies, a specific binding of s4-1BBL or s4-1BBL-Tri_{XVIII}^{LL} to 4-1BB-expressing cells could be confirmed, whereas these soluble proteins did not bind to control cells not expressing 4-1BB. The binding was dose-dependent and still detectable when using a 1:1600 dilution of both supernatants. A control protein that contains a Strep-Tag sequence (in this case Pembrolizumab-scFv), which was used as an additional control, did not bind to 4-1BB-expressing cells and to the control cells.

### 3. s4-1BBL-Tri_{XVIII}^{LL}, s4-1BBL-Tri_{XVIII} and s4-1BBL-Tri_{XV}

The binding of s4-1BBL-TriX_{VIII}^{LL}, s4-1BBL-Tri_{XVIII} and s4-1BBL-Trixvcan be seen in **Figure 4B****.** By performing flow cytometry based binding studies, a specific binding of all three trimerized s4-1BBL polypeptides could be confirmed, whereas these soluble proteins did not bind to control cells not expressing 4-1BB. The binding was dose-dependent for all three trimerized s4-1BBL proteins, but s4-1BBL-Trixv showed by far the weakest binding that decreased fast and was not detectable anymore at a dilution of 1:512. The binding capacities of s4-1BBL-Tri_{XVIII}^{LL} and s4-1BBL-Tri_{XVIII} were very similar and both still detectable at a dilution of 1:4096.

### 4. Triple-s4-1BBL and Triple-s4-1BBL-Tri_{XVIII}^{LL}

The binding of Triple-s4-1BBL and Triple-s4-1BBL-Tri_{XVIII}^{LL} can be seen in **Figure 5B****.** By performing flow cytometry based binding studies, a specific binding of Triple-s4-1BBL and Triple-s4-1BBL-Tri_{XVIII}^{LL} to 4-1BB-expressing cells could be confirmed, whereas these soluble proteins did not bind to control cells not expressing 4-1BB. The binding was dose-dependent for all three trimerized s4-1BBL proteins, but Triple-s4-1BBL-Tri_{XVIII}^{LL} showed by far the weakest binding that decreased quickly and was not detectable at a dilution of 1:256. The polypeptide s4-1BBL-Tri_{XVIII}^{LL} still showed the highest binding capacity even though Triple-s4-1BBL binding was still detectable at a 1:4096 dilution of the supernatant.

### 5. s4-1BBL-TNC

The results of s4-1BBL-TNC binding are shown in **Figure 6B****.** For a better comparison of binding capacity, previously used s4-1BBL-Tri_{XVIII}^{LL} was included in the analysis. By performing flow cytometry based binding studies, a specific binding of s4-1BBL-TNC to 4-1BB-expressing cells could be confirmed, whereas these soluble proteins did not bind to control cells not expressing 4-1BB. The binding was dose-dependent and still detectable when using a 1:128 dilution of the supernatant. Nevertheless, the binding capacity was much lower than for the comparison polypeptide s4-1BBL-Tri_{XVIII}^{LL}, which showed specific binding down to a dilution of 1:4096.

All polypeptides of the present invention had superior performance compared to antibody-based polypeptides as available. Additionally, the specific polypeptide design allows the provision of concise and versatile polypeptides, which can be designed in a way completely avoiding the need of non-human sequences to reduce the risk of side effects during therapeutic applications.

### Example 3: Functional assay

For functional assays, T cell stimulator cells (abbreviated as "TCS") were used to activate the reporter cells. TCS are a murine thymoma cell line (BW5147) engineered to stably express a membrane bound anti-human CD3 single chain fragment (anti-CD3-scFv) that delivers signal one to T cell reporter cells (abbreviation for: TCS-Ctrl) (Leitner et al., 2010). In addition, TCS transduced to express 4-1BBL (TCS-4-1BBL), were used to co-deliver signal one and a co-stimulatory signal to the 4-1BB-expressing reporter cells. This stimulatory effect of 4-1BB can then be easily measured via flow cytometry through an enhanced expression of the NF-κB-reporter gene eCFP, which was mainly used as readout for 4-1BB signaling. The expression levels of 4-1BB on Jurkat reporter cells and the expression of 4-1BBL and anti-CD3-scFv on TCS cells were confirmed using the following antibodies: 4-1BB-PE (4B4-1), 4-1BBL-PE (5F4) (both from Biolegend, San Diego, CA) and a DyLight-649-conjugated goat-anti-mouse IgG(H+L) antibody (Jackson ImmunoResearch, West Grove, PA) for anti-CD3 single chain fragment detection.

5x10⁴ 4-1BB-expressing and control T cell reporter cells were incubated with 2x10⁵ TCS-Ctrl or TCS-4-1BBL. The supernatants comprising the expressed polypeptides (as positive control an agonistic 4-1BB antibody (MAb2) was used at a concentration of 1 µg/ml) were added to the reporter cells that were stimulated with TCS-Ctrl in different concentrations.

For this, 96-well flat bottom plates were used with an end volume of 100 µl. After a 24 hours incubation time NF-κB activation was measured through eCFP expression via flow cytometry. For analysis, TCS cells were excluded by using an APC-conjugated mouse CD45.2 antibody (Biolegend, San Diego, CA) and gating on the APC-negative cell population. Flow cytometry was performed on a FACSFortessa flow cytometer (Becton Dickinson Immunocytometry System, San Jose, CA), using the FACSDiva software. Data was analyzed with FlowJo (version 10.0.7, Tree Star, Ashland, OR) and GraphPad Prism (version 6, GraphPad Software, Inc., La Jolla, CA). The following results were obtained:

### 1. MAb1-scFV and MAb2 ScFv

The results of the functional assay with the supernatants containing MAb1-scFv and MAb2-scFv are shown in **Figure 1D** and **2C****,** respectively. Functional assays, using MAb1-scFv and MAb1-Tri_{XVIII}-scFv supernatants on 4-1BB-expressing reporter cells in addition to TCS-Ctrl, revealed a rather weak capacity of both to functionally activate 4-1BB induced stimulation. Compared to supernatants containing MAb1-scFv the stimulatory capacity of supernatants containing MAb1-Tri_{XVIII}-scFv was stronger. The stimulatory capacity of both supernatants was highest at a final dilution of 1:4. By contrast adding MAb2-scFv or MAb2-scFv-Tri_{XVIII} in supernatants derived from cells transfected with plasmids encoding MAb2-scFv or MAb2-scFv-Tri_{XVIII} to 4-1BB-expressing reporter cells in addition to TCS-Ctrl did not lead to a further stimulation, whereas TCS-4-1BBL was able to enhance NF-κB activation approx. 2.5-fold. As an additional positive control MAb2 (Creative Biolabs, NY) was used, which also enhanced NF-κB activation approx. 1.5-fold. As already stated under Example 2.1, no bands were detected for MAb2 in the Western Blot which correlates with the finding that no functional binding of the MAb2-scFv polypeptides could be confirmed.

### 2. s4-1BBL and s4-1BBL-Tri_{XVII}

The results of the functional assay with the supernatants containing s4-1BBL and s4-1BBL-Tri_{XVIII}^{LL} are shown in **Figure 3C****.** Functional assays, using s4-1BBL or s4-1BBL-Tri_{XVIII}^{LL} supernatants on 4-1BB-expressing reporter cells in addition to TCS-Ctrl, revealed a strong capacity for activating 4-1BB-expressing reporter cells. In particular, the trimerized s4-1BBL was able to functionally activate 4-1BB-induced stimulation. Compared to supernatants containing MAb1-scFv the stimulatory capacity of supernatants containing s4-1BBL-Tri_{XVIII}^{LL} was much stronger. The stimulatory capacity of s4-1BBL-Tri_{XVIII}^{LL} supernatant was still detectable at a final dilution of 1:64, whereas the stimulatory capacity of s4-1BBL supernatant was already very weak, but still detectable, at a final dilution of 1:8.

### 3. s4-1BBL-Tri_{XVIII}^{LL}, s4-1BBL-Tri_{XVIII} and s4-1BBL-Tri_{XV}

The results of the functional assay with the supernatants containing s4-1BBL-Tri_{XVIII}^{LL}, s4-1BBL-Tri_{XVIII} and s4-1BBL-Trixv are shown in **Figure 4C****.** Functional assays, using s4-1BBL-Tri_{XVIII}^{LL}, s4-1BBL-Tri_{XVIII}, and s4-1BBL-Trixv supernatants on 4-1BB-expressing reporter cells in addition to TCS-Ctrl, revealed a very good capacity of all three supernatants to functionally activate 4-1BB induced stimulation. Nonetheless, s4-1BBL-Tri_{XVIII}^{LL} and s4-1BBL-Tri_{XVIII} showed a weaker but more stable activation, whereas s4-1BBL-Trixv induced a stronger activation that wore off faster.

### 4. Triple-s4-1BBL and Triple-s4-1BBL-Tri_{XVIII}^{LL}

The results of the functional assay with the supernatants containing Triple-s4-1BBL and Triple-s4-1BBL-Tri_{XVIII}^{LL} are shown in **Figure 5C****.** For comparison purposes, the functional data were generated with s4-1BBL-Tri_{XVIII}^{LL}as comparator. Functional assays, using s4-1BBL-Tri_{XVIII}^{LL}, Triple-s4-1BBL or Triple-s4-1BBL-Tri_{XVIII}^{LL} supernatants on 4-1BB-expressing reporter cells in addition to TCS-Ctrl, revealed a relatively weak capacity of Triple-s4-1BBL to functionally activate 4-1BB induced stimulation. Triple-s4-1BBL-Tri_{XVIII}^{LL}, on the other hand, showed a stimulatory capacity comparable to s4-1BBL-Tri_{XVIII}^{LL}, but the effect wore off slightly faster.

### 5. s4-1BBL-TNC

The results of the functional assay with the supernatants containing s4-1BBL-TNC in comparison with s4-1BBL-Tri_{XVIII}^{LL} are shown in **Figure 6C****.** Functional assays, using s4-1BBL-TNC or s4-1BBL-Tri_{XVIII}^{LL} supernatants on 4-1BB-expressing reporter cells in addition to TCS-Ctrl, revealed an acceptable capacity of s4-1BBL-TNC to functionally activate 4-1BB induced stimulation. Nonetheless, compared to supernatants containing s4-1BBL-Tri_{XVIII}^{LL} the stimulatory capacity of s4-1BBL-TNC was, even though comparable in higher concentrations, much weaker at lower concentrations. Whereas s4-1BBL-Tri_{XVIII}^{LL} was still at highest stimulatory capacity at a dilution of 1:8, s4-1BBL-TNC at the same concentration already lost about 50% of its effect.

### Example 4:

Two of the polypeptides expressed in the vector pCEP4 of Examples 1 to 3 were cloned into a measles vector for obtaining an oncolytic virus with either MAb1-scFv as payload or s4-1BBL-Tri_{XVIII}^{LL}. A schematic representation of the measles vector and the position of the functional polypeptide can be seen in **Figure 7****.**

### 1. MAb1-scFv in measles vector

The sequence of the monomeric MAb1-based anti-4-1BB scFv was subcloned into vector pc3MerV2ld via Mlul restriction sites. The pc3MerV2ld vector contains an additional transcription unit before the N protein of the Schwarz MV genome (Noll et al., 2013, Int J Oncol. 2013 Jul;43(1):103-12). The generated plasmid was subjected to insert-sequencing using classical Sanger DNA sequencing services. A plasmid with the correct insert in correct orientation and free of mutations was used for virus rescue.

The virus rescue, in the present case, was conducted in Vero cells that were co-transfected with the scFv-based pc3MerV2ld vector and plasmids that lead to production of the measles virus proteins N, P, and L. This results in the formation of infectious measles virus particles that encode the MAb1-based anti-4-1BB scFv, which can be further isolated, purified, and produced (see WO2012/022495).

### 2. s4-1BBL-Tri_{XVIII}^{LL} in measles vector

The nucleic acid sequence of the s4-1BBL-Tri_{XVIII}^{LL} polypeptide (SEQ ID NO: 73) was subcloned into vector pc3MerV2ld via Mlul restriction sites. The pc3MerV2ld vector contains an additional transcription unit before the N protein of the Schwarz MV genome (Noll et al., 2013, *supra*). The generated plasmid was subjected to insert-sequencing using classical Sanger DNA sequencing services. A plasmid with the correct insert in correct orientation and free of mutations was used for virus rescue.

### Example 5: OV cell culture supernatants in binding and functional assays

OV cell culture supernatants and/or supernatants from transfected HEK293 cells can be used to stimulate primary cells, wherein stimulation may be confirmed by measuring cytokine secretion, or by performing proliferation assays. As subsequent step, an *in vivo* confirmation in humanized mouse models (alternative: mouse-specific polypeptides) can be performed. Depending on the nature of the OV of interest, other animal models and/or even prokaryotic test systems can be used.

### Example 6:

The costimulatory polypeptide of the present invention can be used in different OV backbones as payload. For evaluating the general applicability, other established OV backbones were considered for testing. To this end, certain costimulatory polypeptide polypeptides of the present invention can be inserted into an adenovirus backbone to have another well-established OV suitable for therapeutic purposes as cargo for the costimulatory 4-1BBL polypeptides as relevant payload. Exemplary nucleic acid sequences encoding such polypeptides are shown in SEQ ID NOs: 23, 27, 33, 38, 45, 46, 85, 115 and 119. Similar combinations of an OV and the polypeptides of the present invention can be designed for an OV of interest based on the teaching on the costimulatory polypeptides of this invention to obtain an expanded panel of OVs for increasing the therapeutic scope.

## Claims

1. A costimulatory polypeptide comprising
(a)
(i) at least one 4-1BBL ectodomain and a trimerization domain, and/or
(ii) at least one 4-1BBL ectodomain and at least one leader sequence, and optionally at least one affinity tag;
wherein each 4-1BBL ectodomain comprises or consists of the minimal Tumor Necrosis Factor (TNF) Homology Domain according to SEQ ID NO: 1, or a sequence having at least 90%, 91%, 92%, 93%, 94%, 95%, 96%, 97%, 98% or 99% sequence identity to SEQ ID NO: 1 or a nucleic acid sequence encoding the polypeptide;
and/or
(b) at least three 4-1BBL ectodomains, and optionally comprising at least one trimerization domain, wherein each 4-1BBL ectodomain comprises or consists of the minimal TNF Homology Domain according to SEQ ID NO: 1, or a sequence having at least 90%, 91%, 92%, 93%, 94%, 95%, 96%, 97%, 98% or 99% sequence identity to SEQ ID NO: 1, or a nucleic acid sequence encoding the polypeptide; and
wherein the 4-1BBL ectodomain does not comprise the cysteine residue at position 51 of the naturally occurring human 4-1BBL ectodomain as shown in SEQ ID NO: 2, or a nucleic acid sequence encoding the same; and
wherein the polypeptide binds to 4-1BB on the surface of a 4-1BB expressing cell and thus triggers 4-1BB-mediated immune cell stimulation.

2. The costimulatory polypeptide of claim 1, wherein the trimerization domain is selected from SEQ ID NO: 7 and SEQ ID NO: 8.

3. The costimulatory polypeptide of claim 1, wherein the 4-1BBL ectodomain comprises or consists of a sequence according to SEQ ID NOs: 3 to 6 or a polypeptide with a sequence having at least 90%, 91%, 92%, 93%, 94%, 95%, 96%, 97%, 98% or 99% sequence identity to the respective sequence, or a nucleic acid sequence encoding the same.

4. The costimulatory polypeptide of any one of the preceding claims, wherein the polypeptide comprises at least one linker, wherein the at least one linker is individually selected from the group consisting of SEQ ID NOs: 9 to 16, or a sequence having at least 90%, 91%, 92%, 93%, 94%, 95%, 96%, 97%, 98% or 99% sequence identity to any one of the sequences SEQ ID NOs: 9 to 16, or a nucleic acid sequence encoding the same, and/or wherein the costimulatory polypeptide, or the sequence encoding the same, additionally comprises at least one affinity tag and/or at least one protease cleavage tag and/or at least one inhibitory domain and/or at least one leader sequence.

5. The costimulatory polypeptide of any one of the preceding claims, wherein the costimulatory polypeptide is encoded by a nucleic acid sequence selected from the group consisting of SEQ ID NOs: 47, 49, 51, 53, 55, 57, 59, 61, 63, 65, 67, 69, 71, 73, 75, 77 or 79 or a sequence having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to any one of the sequences SEQ ID NOs: 47, 49, 51, 53, 55, 57, 59, 61, 63, 65, 67, 69, 71, 73, 75, 77 or 79.

6. A vector comprising a nucleic acid sequence encoding a costimulatory polypeptide according to any one of the preceding claims.

7. The vector according to claim 6, wherein the vector comprises a sequence encoding an expression vector, or an oncolytic virus, wherein the oncolytic virus is selected from the group consisting of a vaccinia virus, a myxoma virus, an adenovirus, a herpes virus, a reovirus, a Zika virus, a vesicular stomatitis virus, a parvovirus, a poliovirus, an influenza virus, an arenavirus, a coxsackie virus, a semliki forest virus, a sindbis virus, a maraba virus, a seneca valley virus, a Newcastle disease virus, a mumps virus, and a measles virus, or combinations or a chimera thereof.

8. An oncolytic virus encoded by a vector as defined in claim 6, wherein the oncolytic virus comprises a costimulatory polypeptide as payload according to any one of claims 1 to 5.

9. A method of producing a costimulatory 4-1BBL ectodomain polypeptide, the method comprising:
(i) providing a vector according to claim 6 or 7 encoding a costimulatory 4-1BBL polypeptide according to any one of claims 1 to 5 and introducing the vector into a host cell;
(ii) culturing the cell under conditions suitable for expression of the costimulatory 4-1BBL polypeptide according to any one of claims 1 to 5;
(iii) optionally: isolating and purifying the costimulatory 4-1BBL polypeptide; and
(iv) obtaining the costimulatory 4-1BBL polypeptide .

10. A method of producing an oncolytic virus expressing a costimulatory 4-1BBL ectodomain polypeptide according to claim 8, the method comprising:
(i) providing a vector according to claim 7 encoding an oncolytic virus and the costimulatory 4-1BBL ectodomain polypeptide and introducing the vector into a host cell,
(ii) culturing the cell under conditions suitable for expression and thus replication of the oncolytic virus,
(iii) optionally: rescuing the oncolytic virus;
(iv) optionally: purifying the oncolytic virus as obtained in step (ii) or (iii); and
(v) obtaining an oncolytic virus.

11. A pharmaceutical composition comprising a costimulatory polypeptide according to any one of claims 1 to 5 and/or an oncolytic virus according to claim 8, and/or as obtained according to claim 10.

12. A costimulatory polypeptide according to any one of claims 1 to 5, and/or an oncolytic virus according to claim 8, and/or as obtained according to claim 10 for use in a method of treating cancer.

13. The costimulatory polypeptide and/or the oncolytic virus for use in a method of treating cancer according to claim 12, wherein the cancer is selected from bladder cancer, breast cancer, prostate cancer, basal cell carcinoma, biliary tract cancer, bone cancer, brain and central nervous system cancer (e.g., glioma), adenocarcinomas, lung cancer, cervical cancer, choriocarcinoma, colon and rectum cancer, connective tissue cancer, cancer of the digestive system; cancer of the small intestine and cecum; endometrial cancer, esophageal cancer; eye cancer; cancer of the head and neck; gastric cancer; intra-epithelial neoplasm; kidney cancer; larynx cancer; leukemia; liver cancer; gall bladder cancer lung cancer (e.g., small cell and non-small cell); lymphoma including Hodgkin's and Non-Hodgkin's lymphoma; melanoma; myeloma, neuroblastoma, oral cavity cancer (e.g., lip, tongue, mouth, and pharynx); cancer of the salivary glands; ovarian cancer; pancreatic cancer, retinoblastoma; rhabdomyosarcoma; rectal cancer, renal cancer, cancer of the respiratory system; sarcoma, skin cancer; stomach cancer, testicular cancer, thyroid cancer; uterine cancer, cancer of the urinary system, as well as other carcinomas and sarcomas.

14. The costimulatory polypeptide according to any one of claims 1 to 5 or the oncolytic virus according to claim 8 for use in a method of treating cancer according to claim 13, wherein the cancer is a cold tumor or a hot tumor.

15. A kit comprising a costimulatory polypeptide according to any one of claims 1 to 5, or an oncolytic virus according to claim 8, optionally wherein the kit comprises further reagents.

16. Use of an effective amount of costimulatory polypeptide according to any one of claims 1 to 5, or use of an effective amount of oncolytic virus according to claim 8, in an *in vitro* method for stimulating 4-1BB expressing cells.
